# EUROPEAN PATENT SPECIFICATION

(11) **EP 3 027 633 B1**
(45) Date of publication and mention of the grant of the patent: **22.01.2020**
(21) Application number: 14832930.3
(22) Date of filing: 14.07.2014
(51) Int. Cl.: C07H 1/08, A61K 31/7028, A61K 31/7042, A61K 31/70, A61P 3/00, A61P 3/06, A61P 9/00, A61P 21/00, A61K 31/7048

(54) **ULMOSIDE-A FOR PREVENTION OR TREATMENT OF ADIPONECTIN DEPLETION ASSOCIATED METABOLIC DISORDERS**
ULMOSID-A ZUR PRÄVENTION ODER BEHANDLUNG DER MIT ADIPONEKTINSCHWUND VERBUNDENE STOFFWECHSELKRANKHEITEN
ULMOSIDE-A POUR LA PRÉVENTION OU LE TRAITEMENT DES MALADIES MÉTABOLIQUES ASSOCIÉES À LA RÉDUCTION DE L'ADIPONECTINE

(30) Priority: 02.08.2013 IN 2326DE2013
(43) Date of publication of application: 08.06.2016
(73) Proprietor: Council of Scientific and Industrial Research, New Delhi 110001 (IN)
(72) Inventor: SANYAL, Sabyasachi, Lucknow 226001 (IN); CHATTOPADHYAY, Naibedya, Lucknow 226001 (IN); MAURYA, Rakesh, Lucknow 226001 (IN); GAYEN, Jiaur Rahaman, Lucknow 226001 (IN); BHADAURIA, Smrati, Lucknow 226001 (IN); TRIVEDI, Arun Kumar, Lucknow 226001 (IN); SINGH, Abhishek Kumar, Lucknow 226001 (IN); MISHRA, Jay Sharan, Lucknow 226001 (IN); KUMARI, Rashmi, Lucknow 226001 (IN); SHARAN, Kunal, Lucknow 226001 (IN); KHAN, Parvez Mohammad, Lucknow 226001 (IN); KHAN, Kainat, Lucknow 226001 (IN); SINGH, Nidhi, Lucknow 226001 (IN); DWIVEDI, Shailendra Kumar Dhar, Lucknow 226001 (IN); YADAV, Manisha, Lucknow 226001 (IN); DIXIT, Preety, Lucknow 226001 (IN); MISHRA, Devendra Pratap, Lucknow 226001 (IN); SHARMA, Sharad, Lucknow 226001 (IN); KAMAL, Ram Arya, Lucknow 226001 (IN)
(74) Representative: AWA Sweden AB
(86) International application number: PCT/IN2014/000464
(87) International publication number: WO 2015/015509

(56) References cited:
- US-A1- 2006 177 528
- US-A1- 2008 131 534
- US-A1- 2009 234 150
- US-A1- 2009 312 246
- US-A1- 2011 112 042
- US-A1- 2012 121 530
- GAURAV SWARNKAR ET AL: "A novel flavonoid isolated from the steam-bark of Ulmus Wallichiana Planchon stimulates osteoblast function and inhibits osteoclast and adipocyte differentiation", EUROPEAN JOURNAL OF PHARMACOLOGY, vol. 658, no. 2-3, 1 May 2011 (2011-05-01) , pages 65-73, XP055346653, NL ISSN: 0014-2999, DOI: 10.1016/j.ejphar.2011.02.032
- KUNAL SHARAN ET AL: "A novel quercetin analogue from a medicinal plant promotes peak bone mass achievement and bone healing after injury and exerts an anabolic effect on osteoporotic bone: The role of aryl hydrocarbon receptor as a mediator of osteogenic action", JOURNAL OF BONE AND MINERAL RESEARCH, vol. 26, no. 9, 19 August 2011 (2011-08-19), pages 2096-2111, XP055346660, US ISSN: 0884-0431, DOI: 10.1002/jbmr.434

## Description

### FIELD OF THE INVENTION

The present invention relates to a method for treatment or prevention of adiponectin depletion-associated metabolic disorders by using Ulmoside-A. The present invention further relates to use of Ulmoside-A (ULMA- Molecular Wt. 460) ((2S,3S)-(+)-3',4',5,7-tetrahydroxydihydroflavonol-6-*C*-β-D-glucopyranoside as a novel small molecule ligand for adiponectin receptors for alleviation, management or prevention or treatment of steroid-induced metabolic disorder. More particularly, the present invention relates to a pharmaceutical composition useful for prevention or treatment of various medical indications associated with metabolic diseases caused in humans and animals associated with low plasma adiponectin.

### BACKGROUND OF THE INVENTION

The *Ulmus wallichiana* Planchon, belongs to family Ulmaceae, is a large deciduous tree and distributed through Himalayas from Afghanistan to W. Nepal [Dictionary of Indian Folk Medicine and Ethnobotany edited by Jain, S. K., Deep Publications, Paschim Vihar, New Delhi, India, 1991, pp183]. Leaves of the plant yield fodder and bark yield strong fiber. In India, this plant is found in Kumaon and Garhwal Himalaya, locally called as Chamarmou, is a deciduous tree growing to 35m in height. Previously we investigated plant extract and fractions pharmacologically, which showed nonestrogenic osteoprotective effect [K. Sharan, J.A. Siddigui, G. Swarnkar, A. M. Tyagi, A. Kumar, P. Rawat, M. Kumar, G. K. Nagar, K. R. Arya, L. Manickayasagam, G. K. Jain, R. Maurya, N. Chattopadhyay, Menopause 17 (2); 393-402. 2010]. Moreover chemical investigation of bark of *Ulmus wallichiana* planchon, resulted in isolation of eighth pure compound [P. Rawat, M. Kumar, K. Sharan, N. Chattopadhyay, R. Maurya, Bioorg Med Chem Lett 19; 4684-4687, 2008]. The isolated new compound, ULMA mitigated ovariectomy- induced osteoporosis in rats [K. Sharan, , G. Swarnkar, J. K. Siddigui, A. Kumar, P. Rawat, M. Kumar, G. K. Nagar, L. Manickayasagam, S. P. Singh, G. Mishra, Wahajuddin, G. K. Jain, R. Maurya, N. Chattopadhyay, Menopause 17 (3); 577-586. 2010; R. Maurya, P. Rawat, K. Sharan, J. K. Siddigui, G. Swarnkar, G. Mishra, L. Manickayasagam, G. K. Jain, K. R. Arya, N. Chattopadhyay, [WO 2009/110003]. Phenolic and flavonoid C-glycosides including ULMA have been evaluated for antihyperglycemic activity in acute treatment situation [P. Rawat, M. Kumar, N. Rahuja, D. S. L. Srivastava, A. K. Srivastava, R. Maurya. Bioorganic & Medicinal Chemistry Letters 21; 228-233, 2011].

ULMA is a flavonoid C-glycoside. O-glycosylation is a common metabolic fate for majority of flavonoids, an event that is also known to influence their stability. For example, rutin (quercetin- 3-O-glucose rhamnose), distributed in many plants, dietary glycosides, are converted to aglycones, such as quercetin, in the large intestine in reactions catalyzed by the glycosidase of intestinal bacteria [G. Tamura, C. Gold, A. Fezz-Luzi, B. N. Ames. Proc. Natl. Acad. Sci. U.S.A. 77; 4961,1999]. Rutin inhibits the ovariectomy-induced resorption of bone in rats [M.-N. Horcajada-Molteni, V. Crespy, V. Coxam, M.-J. Davicco, C. Remesy, J.-P. Barlet. J. Bone Miner. Res. 15; 2251, 2000] and quercetin has been reported to inhibit the osteoclastic resorption of bone in vitro [A. Wattel, S. Kamel, R. Mentaverri, F. Lorget, C. Prouillet, J.-P. Petit, P. Fardelonne, M. Brazier. Biochem. Pharmacol. 65; 35. 2003; M. Notoya, Y. Tsukamoto, H. Nishimura, J.-T. Woo, K. Nagai, I.-S. Lee, H. Hagiwara. Eur. J. Pharmacol. 485; 89, 2004]. So far, there is no report on C-glycosylated flavonoids for their potential in metabolic disorders. We hypothesized that C-glycosylated flavonoids will be better therapeutic candidates given their stability over aglycone or O-glycosylated flavonoids.

Adipoenctin is an anti-inflammatory cytokine produced mainly by adipose tissue (T. Kadowaki, T. Yamauchi, N. Kubota, K. Hara, K. Ueki, K. Tobe. J Clin Invest. 116(7);1784-92. 2006.). Adiponectin signaling is primarily mediated through adiponectin receptors 1 and 2 (AdipoRl and AdipoR2), two unique plasma membrane receptors with 7 trans-membrane domains that have extracellular C-termini and are not known to be coupled to any G-protein (T. Kadowaki, T. Yamauchi, N. Kubota, K. Hara, K. Ueki, K. Tobe. J Clin Invest. 116(7); 1784-92. 2006.). T-cadherin, a unique member of the cadherin family that lacks a transmembrane and cytoplasmic domain was also identified as an adiponectin binding protein although whether it supports any adiponectin signalling is not known (T. Kadowaki, T. Yamauchi, N. Kubota, K. Hara, K. Ueki, K. Tobe. J Clin Invest. 116(7);1784-92. 2006.). Over the years a number of studies have demonstrated the importance of adiponectin in number of metabolic disorders including insulin resistance, obesity, metabolic syndrome, vascular and cardiac pathophysiology as well as cancer (T. Kadowaki, T. Yamauchi, N. Kubota, K. Hara, K. Ueki, K. Tobe. J Clin Invest. 116(7);1784-92. 2006.; S. Dridi, M. Taouis. Journal of Nutritional Biochemistry 20 (2009) 831-839. 2009; M. Dalamaga, K.N. Diakopoulos, C.S. Mantzoros. Endocr Rev; 33(4):547-94. 2012).

Genetic evidences from human studies have established that adiponectin locus 3q27 belongs to one of the 3 loci that is a determinant for susceptibility to insulin resistance across multiple ethnic populations (T. Kadowaki, T. Yamauchi, N. Kubota, K. Hara, K. Ueki, K. Tobe. J Clin Invest. 116(7);1784-92. 2006). Clinical studies have established that reduction in adiponectin level can be single most important marker for insulin resistance(T. Kadowaki, T. Yamauchi, N. Kubota, K. Hara, K. Ueki, K. Tobe. J Clin Invest. 116(7);1784-92. 2006; S. Dridi, M. Taouis. J Nutr Biochem 20 (2009) 831-839. 2009). Ectopic expression or treatment with adiponectin has been found to ameliorate insulin resistance, skeletal muscle atrophy, metabolic syndrome and atherosclerosis in a substantial number of animal studies (T. Kadowaki, T. Yamauchi, N. Kubota, K. Hara, K. Ueki, K. Tobe. J Clin Invest. 116(7); 1784-92. 2006, S. Dridi, M. Taouis. J Nutr Biochem 20 (2009) 831-839. 2009, A.D. Kandasamy, M.M. Sung, J.J. Boisvenue, A.J. Barr, J.R.B Dyck. Nutrition and Diabetes;. 2012; T. Fiaschi, D. Cirelli, G. Comito, S. Gelmini, G. Ramponi, M. Serio, P. Chiarugi. Cell Res. 19(5):584-97.2009).

These evidences from animal experiments are supported by clinical studies where low level of adiponectin has been strongly correlated with insulin resistance, cardiac hypertrophy, alcoholic and non-alcoholic fatty liver diseases, and dysfunctional skeletal muscle bioenergetics (T. Kadowaki, T. Yamauchi, N. Kubota, K. Hara, K. Ueki, K. Tobe. J Clin Invest. 116(7); 1784-92. 2006, S. Dridi, M. Taouis. J Nutr Biochem 20 (2009) 831-839. 2009, A.E. Civitarese, B. Ukropcova, S. Carling, M. Hulver, R.A. DeFronzo, L. Mandarino , E. Ravussin, S.R. Smith. Cell Metab;4(1):75-87. 2006, H. Mitsuhashi, H. Yatsuya, K. Tamakoshi, K. Matsushita, R. Otsuka, K. Wada, K. Sugiura, S. Takefuji, Y. Hotta, T. Kondo, T. Murohara, H. Toyoshima. Hypertension;49:1448-1454. 2007. , M. Iwabu, T. Yamauchi, M. Okada-Iwabu, K. Sato, T. Nakagawa, M. Funata, M.. Yamaguchi, S. Namiki, R. Nakayama, M. Tabata, H. Ogata, N. Kubota, I. Takamoto, Y. K. Hayashi, N. Yamauchi, H. Waki, M. Fukayama, I. Nishino, K. Tokuyama, K. Ueki, Y. Oike, S. Ishii, K. Hirose, T. Shimizu, K. Touhara, T. Kadowaki. Nature; 464(7293):1313-1319. 2010, M. You,C.Q. Rogers. Exp Biol Med; 234 (8) 850-859. 2009). Further, adiponectin, Beta-acting through AdipoR2 enhances pancreatic beta cell survival (N. Wijesekara, M. Krishnamurthy, A. Bhattacharjee, A. Suhail, G. Sweeney, M.B. Wheeler. J Biol Chem; 285, 33623-33631. 2010).

The above evidences make AdipoRl and R2 extremely important therapeutic targets for a number of metabolic diseases/disorders including insulin resistance, type 2 diabetes, skeletal muscle atrophy, cardiovascular diseases, such as cardiac hypertrophy, cardiomyopathy, myocardial infarction, atherosclerosis, alcoholic and non-alcoholic fatty liver diseases, pancreatic beta cell degeneration, type I diabetes and cancer.

The major physiological outcomes of adiponectin signalling have been attributed to its role in enhancement of skeletal muscle mitochondrial function and fatty acid oxidation (T. Kadowaki, T. Yamauchi, N. Kubota, K. Hara, K. Ueki, K. Tobe. J Clin Invest. 116(7);1784-92. 2006). Adiponectin achieves this primarily via regulation of expression of fatty acid transporters {cluster of differentiation 36 (CD36), fatty acid binding protein 3 (Fabp3)}, enzymes involved in fatty acid β-oxidation {acetyl coenzyme A carboxylase (ACC), acetyl coenzyme oxidase 1 (ACOX1), carnitine palmitoyl transferase 1β (CPT1β), fatty acyl coenzyme A synthetase} mitochondrial uncoupling proteins (uncoupling protein -1, -2 and -3), activation of p38 mitogen activated protein kinase (p38 MAPK), adenosine monophosphate dependent protein kinase (AMPK) and enhancement in expression and activity of peroxisome proliferator activated receptor alpha (PPAR a) and PPAR gamma co-activator 1 alpha (PGC-1α) (T. Kadowaki, T. Yamauchi, N. Kubota, K. Hara, K. Ueki, K. Tobe. J Clin Invest. 116(7);1784-92. 2006, M. Iwabu, T. Yamauchi, M. Okada-Iwabu, K. Sato, T. Nakagawa, M. Funata, M. Yamaguchi, S. Namiki, R. Nakayama, M. Tabata, H. Ogata, N. Kubota, I. Takamoto, Y. K. Hayashi, N. Yamauchi, H. Waki, M. Fukayama, I. Nishino, K. Tokuyama, K. Ueki, Y. Oike, S. Ishii, K. Hirose, T. Shimizu, K. Touhara, T. Kadowaki. Nature; 464(7293):1313-1319. 2010). Also adiponectin has been shown to enhance uncoupling protein 1 (UCP-1) in adipose tissue depots which may contribute to enhanced fatty acid oxidation in adipose tissue depot and improvement of overall lipid profile.

Structurally, adiponectin belongs to the complement 1q family (T. Kadowaki, T. Yamauchi, N. Kubota, K. Hara, K. Ueki, K. Tobe. J Clin Invest. 116(7);1784-92. 2006). Adipoenctin monomer is a 30 KD protein that consists of an N-terminal collagenous domain and a C-terminal globular domain. Mammalian plasma adiponectin is present in several multimeric forms such as low molecular weight dimer or trimers, medium-molecular-weight hexamers or high molecular weight -complexes of dodecamers and 18 mers. The globular fragment, that results from proteolytic cleavage, is also detectable in human or mouse plasma as a trimeric form. All these forms have shown different levels of physiological activity and at present the HMW is considered the mostly clinically relevant form (T. Kadowaki, T. Yamauchi, N. Kubota, K. Hara, K. Ueki, K. Tobe. J Clin Invest. 116(7); 1784-92. 2006). The HMW full-length adiponectin and the globular form have been shown to preferentially signal through AdipoR2 and AdipoRl respectively (T. Kadowaki, T. Yamauchi, N. Kubota, K. Hara, K. Ueki, K. Tobe. J Clin Invest. 116(7);1784-92. 2006). Given the multemerization-related complexities of adiponectin structure and function and also the limitations associated with substantial expenses in industrial scale preparation of biologics, we opined that small molecule regulators of adiponectin receptors may provide the only viable strategy.

A recent report described identification of 9 small molecule adiponectin receptor agonists (Sun Y, Zang Z Zhong L, Wu M, Su Q.et al 2013, PLoS ONE 8(5):2013), however, the compounds described in this study have no resemblance with ULMA.

Reference may be made to the research article by Sun Y, Zang Z , Zhong L, Wu M,Su Q.et al 2013, PLoS ONE 8(5):2013 wherein the compounds disclosed are adiponectin receptor agonist which are considered to treat hypo-adiponectinemia that is associated with type-2 diabetes, insulin resistance, atherosclerosis, coronary heart disease and malignancies.

Patent application US 2011/0112042 discloses a flavonol compound and a bioactive extract/fraction from Ulmus wallichiana. Patent application US 2008/0131534 relates to methods for increasing peroxisome proliferator-activated receptor-gamma (PPARy) activity and/or endothelial nitiric oxide synthase activity. Patent application US 2009/0312246 relates to use of incretin compounds and amylinomimetic compounds. Patent application US 2009/0234150 relates to muscle pathologies, more particularly to the field of diseases where skeletal muscle damage and muscle loss occurs. Patent application US 2012/0121530 relates to DPP-4 inhibitors. Patent application US 2006/0177528 relates to Free-B-ring flavonoids and flavans. A publication in European Journal of Pharmacology, vol. 658, no. 2-3, 1 May 2011, pages 65-73, relates to flavonoid isolated from the steam-bark of Ulmus Wallichiana Planchon. A publication in Journal of Bone and Mineral Research, vol. 26, no. 9, 19 August 2011, pages 2096-2111, relates to quercetin analogue from a medicinal plant.

In the present invention ULMA has been identified as a novel small molecule modulator of adiponectin receptors

### OBJECTIVE OF THE INVENTION

The main objective of the present invention is to provide Ulmoside-A from *Ulmus wallichiana* as a small molecule (Mol. Wt. 460) agonist for adiponectin receptors 1 (AdipoRl) and 2 (AdipoR2) useful for prevention or cure of metabolic diseases.

Another objective of the present invention is to provide Ulmoside-A (ULMA) ((2S,3S)-(+)-3',4',5,7-tetrahydroxydihydroflavonol-6-*C*-β-D-glucopyranoside) for alleviation, management or prevention or treatment of steroid-induced metabolic disorder.

Still another objective of the present invention is to provide a dosage regimen and a mode of administration of the compound of the present invention with one or more of the pharmaceutically acceptable carrier or excipient etc. The dosage will vary according to the type of disorder, the disease conditions and will be subject to the judgment of the medical practitioner involved.

### Abbreviations

ULMA: Ulmoside-A
AdipoRl: Adiponectin receptor 1
AdipoR2: Adiponectin receptor 2
CD36: cluster of differentiation 36
Fabp3: fatty acid binding protein 3
ACC: Acetyl coenzyme A carboxylase
ACOX1: acetyl coenzyme oxidase 1
CPT1: Carnitine palmitoyl transferase 1
UCP: mitochondrial uncoupling protein
P38MAPK: p38 mitogen activated protein kinase
AMPK : Adenosine monophosphate dependent protein kinase
PPARα: Peroxisome proliferator activated receptor alpha
PGC-1α: PPAR gamma co-activator 1 alpha
PRDM16: PR domain containing 16
Dex: Dexamethasone
BW: Body weight
IP: Intra-peritoneal
Murf1: muscle RING-finger protein-1
Glul: Glutamate ammonia ligase
OGTT: Oral glucose tolerance test

### SUMMARY OF THE INVENTION

According to the present invention, there is provided a compound of formula A or a pharmaceutically acceptable salt thereof for use in treatment or prevention of adiponectin depletion associated metabolic disorders.

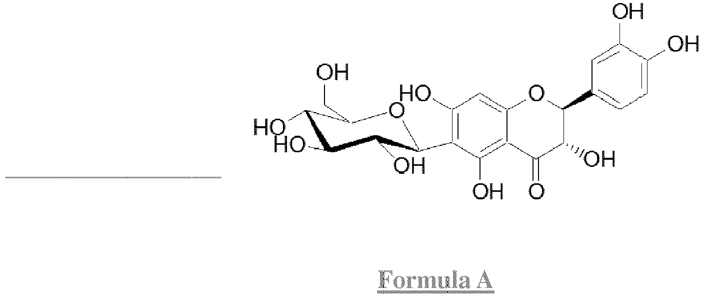

Disclosed herein is a method for treatment or prevention of adiponectin depletion-associated metabolic disorders, the method comprising administering a therapeutically effective amount of a compound of formula A or a pharmaceutically acceptable salt thereof or a composition comprising a compound of formula A and at least one pharmaceutically acceptable carrier or excipient to a subject in need thereof.

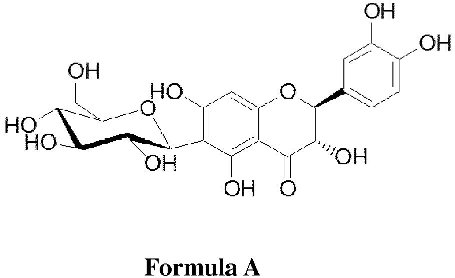

The subject may be a mammal, preferably human.

Further disclosed is a method for treatment or prevention of adiponectin depletion-associated metabolic disorders, wherein the compound of formula A or a pharmaceutically acceptable salt thereof or a composition comprising a compound of formula A and at least one pharmaceutically acceptable carrier or excipient is administered in dose from 0.1 mg to 5000 mg, preferably from 0.5 to 1000, more preferably from 1 mg to 500 mg weekly or bi-weekly or daily or twice a day or three times a day or in still more divided doses.

Further disclosed is a method for treatment or prevention of adiponectin depletion associated metabolic disorders, wherein the compound or composition is administered by a route selected from the group consisting of oral, systemic, local, topical, intravenous, intra-arterial, intra-muscular, subcutaneous, intra-peritoneal, intra-dermal, buccal, intranasal, inhalation, vaginal, rectal and transdermal.

Further disclosed is a method for treatment or prevention of adiponectin depletion associated metabolic disorders, wherein the adiponectin depletion-associated metabolic disorder is selected from the group consisting of steroid-induced metabolic disorders, skeletal muscle atrophy, and cardiac hypertrophy.

Further disclosed is a method for treatment or prevention of adiponectin depletion-associated metabolic disorders, wherein the steroid is selected from the group consisting of dexamethasone, corticosteroid and prednisolone.

Further disclosed is a method for treatment or prevention of adiponectin depletion-associated metabolic disorders, wherein skeletal muscle atrophy is caused by disuse of muscles, denervation, sepsis, fasting or cancer cachexia.

Further disclosed is a method for treatment or prevention of adiponectin depletion associated metabolic disorders, wherein the induced cardiac hypertrophy is selected from the group consisting of neurohormone-mediated hypertrophy, hypoxia-mediated hypertrophy, stress-mediated hypertrophy, myocardial infraction-mediated hypertrophy, hypertension-mediated hypertrophy and drug-induced hypertrophy.

Further disclosed is a method for treatment or prevention of adiponectin depletionassociated metabolic disorders, wherein the compound of formula A or a pharmaceutically acceptable salt thereof or a composition comprising a compound of formula A and at least one pharmaceutically acceptable carrier or excipients is administered in an amount effective to reduce body weight (obesity) or reduce blood glucose in an obese subject.

Further disclosed is a method for treatment or prevention of adiponectin depletion-associated metabolic disorders wherein, the composition of the compound of formula A is in the form of a suspension, liquid formulation, tablet, pill, capsule, powder or granule containing at least one of the following pharmaceutically acceptable excipient:
(i) a diluent selected from the group consisting of lactose, mannitol, sorbitol, microcrystalline cellulose, sucrose, sodium citrate and dicalcium phosphate or a combination thereof;
(ii) a binder selected from the group consisting of gum tragacanth, gum acacia, methyl cellulose, gelatin, polyvinyl pyrrolidone and starch or a combination thereof;
(iii) a disintegrating agent selected from the group consisting of agar-agar, calcium carbonate, sodium carbonate, silicates, alginic acid, corn starch, potato tapioca starch and primogel or a combination thereof;
(iv) a lubricant selected from the group consisting of magnesium stearate, calcium stearate, calcium steorotes, talc, solid polyethylene glycols and sodium lauryl sulphate or a combination thereof;
(v) a glidant such as colloidal silicon dioxide;
(vi) a sweetening agent selected from the group consisting of sucrose, saccharin and fructose or a combination thereof;
(vii) a flavoring agent selected from the group consisting of peppermint, methyl salicylate, orange flavor and vanilla flavor or a combination thereof;
(viii) a wetting agent selected from the group consisting of cetyl alcohol and glyceryl monostearate or a combination thereof;
(ix) an absorbent selected from the group consisting of kaolin and bentonite clay or a combination thereof; and
(x) a solution retarding agent selected from the group consisting of wax and paraffin or a combination thereof.

The present invention provides a compound of formula A or a pharmaceutically acceptable salt thereof for use in treatment or prevention of adiponectin depletion associated metabolic disorders.

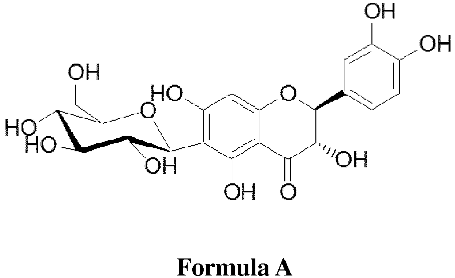

In an embodiment of the present invention, there is provided a compound of formula A or a pharmaceutically acceptable salt thereof, wherein said compound is administered in dose from 0.1 mg to 5000 mg, preferably from 0.5 to 1000, more preferably from 1 mg to 500 mg weekly or bi-weekly or daily or twice a day or three times a day or in still more divided doses.

In another embodiment of the present invention, there is provided a compound of formula A or a pharmaceutically acceptable salt thereof, wherein the compound is administered by a route selected from the group consisting of oral, systemic, local, topical, intravenous, intra-arterial, intra-muscular, subcutaneous, intra-peritoneal, intra-dermal, buccal, intranasal, inhalation, vaginal, rectal and transdermal.

In yet another embodiment of the present invention, there is provided a compound of formula A or a pharmaceutically acceptable salt thereof, wherein the adiponectin depletion associated metabolic disorders is selected from the group consisting of steroid-induced metabolic disorders, skeletal muscle atrophy, induced cardiac hypertrophy and obesity.

In still another embodiment of the present invention, there is provided a compound of formula A or a pharmaceutically acceptable salt thereof, wherein the steroid is selected from the group consisting of dexamethasone, corticosteroid and prednisolone; the skeletal muscle atrophy is caused by disuse of muscles, denervation, sepsis, fasting or cancer cachexia; and the induced cardiac hypertrophy is selected from the group consisting of neurohormone-mediated hypertrophy, hypoxia-mediated hypertrophy, stress-mediated hypertrophy, myocardial infraction-mediated hypertrophy, hypertension-mediated hypertrophy and drug-induced hypertrophy.

The present invention provides a composition comprising a compound of formula A and at least one pharmaceutically acceptable carrier or excipient for use in treatment or prevention of adiponectin depletion associated metabolic disorders.

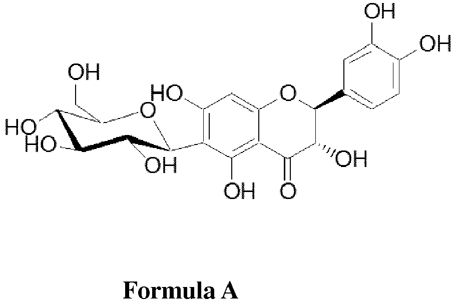

In an embodiment of the present invention, there is provided a composition comprising a compound of formula A and at least one pharmaceutically acceptable carrier or excipient, wherein said composition is administered in dose from 0.1 mg to 5000 mg, preferably from 0.5 to 1000, more preferably from 1 mg to 500 mg weekly or bi-weekly or daily or twice a day or three times a day or in still more divided doses.

In another embodiment of the present invention, there is provided a composition comprising a compound of formula A and at least one pharmaceutically acceptable carrier or excipient, wherein the composition is administered by a route selected from the group consisting of oral, systemic, local, topical, intravenous, intra-arterial, intra-muscular, subcutaneous, intra-peritoneal, intra-dermal, buccal, intranasal, inhalation, vaginal, rectal and transdermal.

In yet another embodiment of the present invention there is provided a composition comprising a compound of formula A and at least one pharmaceutically acceptable carrier or excipient, wherein the adiponectin depletion associated metabolic disorders is selected from the group consisting of steroid-induced metabolic disorders, skeletal muscle atrophy, induced cardiac hypertrophy and obesity.

In still another embodiment of the present invention, there is provided a composition comprising a compound of formula A and at least one pharmaceutically acceptable carrier or excipient, wherein the steroid is selected from the group consisting of dexamethasone, corticosteroid and prednisolone; the skeletal muscle atrophy is caused by disuse of muscles, denervation, sepsis, fasting or cancer cachexia; and the induced cardiac hypertrophy is selected from the group consisting of neurohormone-mediated hypertrophy, hypoxia-mediated hypertrophy, stress-mediated hypertrophy, myocardial infraction-mediated hypertrophy, hypertension-mediated hypertrophy and drug-induced hypertrophy.

Disclosed herein is a use of a compound of formula A or a pharmaceutically acceptable salt thereof in manufacture of a medicament for treatment or prevention of adiponectin depletion associated metabolic disorders.

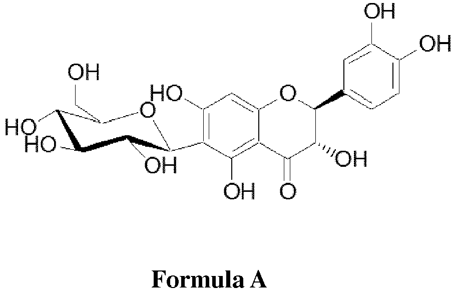

Further disclosed is a use of a compound of formula A or a pharmaceutically salt thereof, wherein the compound is administered in dose from 0.1 mg to 5000 mg, preferably from 0.5 to 1000, more preferably from 1 mg to 500 mg weekly or bi-weekly or daily or twice a day or three times a day or in still more divided doses.

Further disclosed is a use of a compound of formula A or a pharmaceutically acceptable salt thereof, wherein the compound is administered by a route selected from the group consisting of oral, systemic, local, topical, intravenous, intra-arterial, intra-muscular, subcutaneous, intra-peritoneal, intra-dermal, buccal, intranasal, inhalation, vaginal, rectal and transdermal.

Further disclosed is a use of a compound of formula A or a pharmaceutically acceptable salt thereof wherein the adiponectin depletion associated metabolic disorders is selected from the group consisting of steroid-induced metabolic disorders, skeletal muscle atrophy, induced cardiac hypertrophy and obesity.

Further disclosed is a use of a compound of formula A or a pharmaceutically acceptable salt thereof wherein the steroid is selected from the group consisting of dexamethasone, corticosteroid and prednisolone; the skeletal muscle atrophy is caused by disuse of muscles, denervation, sepsis, fasting or cancer cachexia; and the induced cardiac hypertrophy is selected from the group consisting of neurohormone-mediated hypertrophy, hypoxia-mediated hypertrophy, stress-mediated hypertrophy, myocardial infraction-mediated hypertrophy, hypertension-mediated hypertrophy and drug-induced hypertrophy.

Further disclosed is a use of composition comprising a compound of formula A and at least one pharmaceutically acceptable carrier or excipient for treatment or prevention of adiponectin depletion associated metabolic disorders.

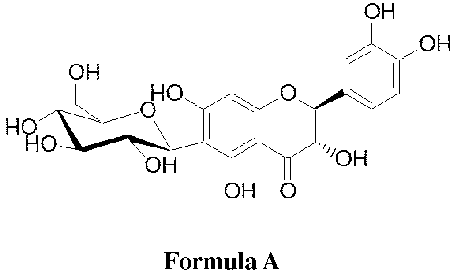

### BRIEF DESCRIPTION OF THE ACCOMPANYING DRAWINGS

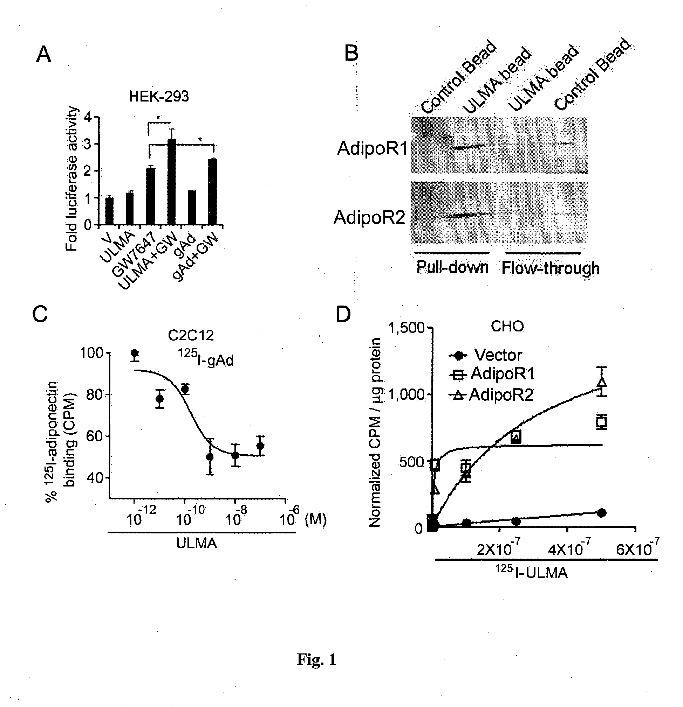

**Fig. 1****: ULMA activates adiponectin receptor and shows direct binding with AdipoRl and AdipoR2.**
   **A.** ULMA enhances PPARα ligand activity; HEK293 cells were seeded on to 24 well plates and after 24h, transfected with 100ng of GAL-PPARα and 100ng GAL4-Luc along with 100ng eGFPC1 (Clontech) plasmids using lipofectamine LTX (Invitrogen) according to manufacturer's instructions. 24h after transfection, cells were treated with or without 100pM GW-7647 alone or in combination with 100nM ULMA or 1µ/ml globular adiponectin (gAd) for 6h. Following which, the cells were lysed and GFP fluorescence and firefly luciferase activities were measured. Luciferase activity was normalized with GFP fluorescence and plotted as fold activity over vehicle (0.1% DMSO) treated control. Data shown in mean ± SEM of three independent experiments performed in duplicates. *p<0.05 as determined by two tailed unpaired student's t-test.
   **B.** ULMA interacts with AdipoRs. 20 µl of ULMA beads or control beads were incubated with 50 µg of plasma membrane extracts (prepared using a membrane protein isolation kit; Biomol; according to manufacturer's instructions) prepared from C2C12 myotubes (that expresses both AdipoRl and AdipoR2) for 12h on a rotary wheel set at 10 rpm. The supernatant was removed and an aliquot was stored as flowthrough. The pellet was washed 6 times and then the beads were boiled in Lamelli buffer and resolved by 10% denaturing polyacrylamide gel electrophoresis followed by transfer on nitrocellulose membrane and western blotting with anti AdipoRl or anti-AdipoR2 antibodies. Data represents one of two independent experiments showing identical pattern.
   **C.** ULMA competes with ¹²⁵I- globular adiponectin (gAd) for binding to AdipoRs. C2C12 myotubes in 12 well plates were incubated with 2µl of 10uCi/ml ¹²⁵I-adiponectin (this amount gives 50% binding to the cells) in ice-cold PBS and 0.1% bovine serum albumin for 12h in presence or absence of different doses of unlabeled ULMA (10⁻¹¹ M, 10⁻¹⁰ M, 10⁻⁹ M, 10⁻⁸ M, 10⁻⁷ M, 10⁻⁶M) at 4°C. The cells were then washed 20 times in PBS and then lysed. 5µl of the lysate was used for protein estimation using standard Bradford assay and rest of the lysate was used for measuring radioactivity in a gamma-counter (Cole Palmer). The count per minute was normalized with protein concentration and plotted as % binding compared to wells treated with vehicle only (0.1% vol/vol DMSO). Data represents mean ± SEM from three experiments performed in triplicates.
   **D.** CHO cells transfected with empty vector or AdipoRl or R2 expression vectors were incubated with various concentrations of ¹²⁵I-ULMA for 2h at 4°C. Following washes, cells were lysed and count per minute was analyzed in a gamma counter. 200 fold molar excess of unlabeled ULMA was used for each concentration of radiolabeled ULMA for determining nonspecific activity. Count per minutes were normalized with total cellular protein content followed by further normalization with non-specific activity and plotted. Data is mean± SEM from three experiments performed in triplicates.

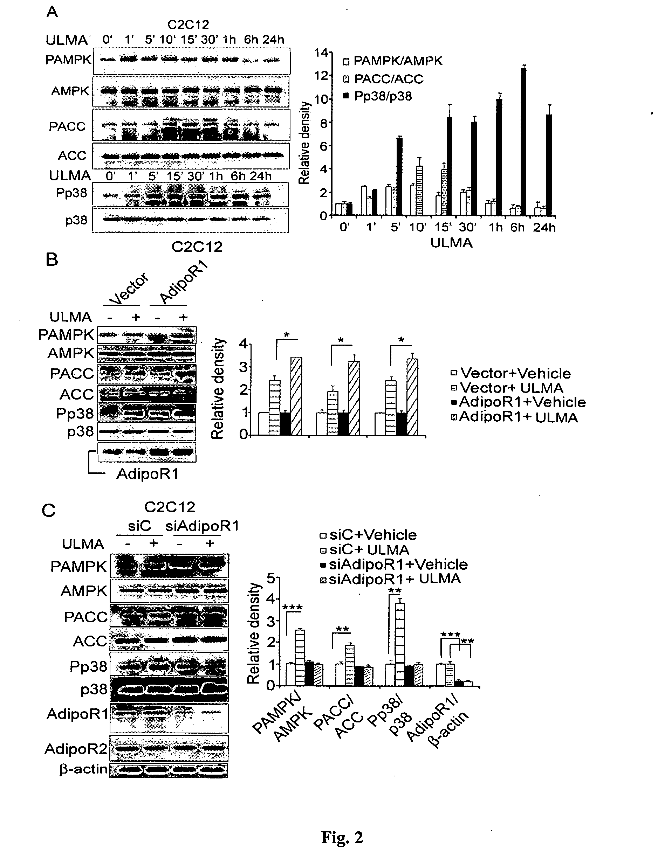

**Fig. 2****: ULMA induces downstream signaling of adiponectin pathway and AdipoR knockdown mitigates its activity.**
   **A.** ULMA induces rapid AMPK and p38MAPK signaling. C2C12 myotubes in 10 cm dishes were treated with vehicle (0.1% vol/vol DMSO; 0 min) or 10 nM ULMA for different time points ranging from 1 min to 24h (final concentration of DMSO in all wells were 0.1% vol/vol). Following treatment, the cells were washed with ice-cold PBS and then lysed. The total protein was estimated by Bradford assay and equal amount of protein (50 µg) was resolved by denaturing polyacrylamide gel electrophoresis and western blotted to determine pAMPK, pACC and pP38 level. Total AMPK, total ACC or total P38 levels were used as loading controls. Data is representative of three independent experiments displaying identical pattern (right panel depicts densitometry from 3 experiments).
   **B.** AdipoRl overexpression enhances ULMA effect on AMPK and p38 MAPK signaling. C2C12 myoblasts growing in T75 flasks were trypsinized and transfected with 10µg of either empty vector (pcDNA3) or AdipoRl expression plasmid using lipofectamine LTX transfection reagent and then the cells were plated in 10 cm dishes. 24h following transfection, cells were incubated in differentiation medium and maintained in the same medium for 96h when the cells differentiated fully into myotubes. These cells were then treated with vehicle (DMSO) or ULMA (10 nM) for 10 mins. The cells were then lysed and western blotted for the indicated proteins. Data is representative of three independent experiments displaying identical pattern. (Right panel shows densitometry of three experiments). *p<0.05 as determined by two tailed unpaired student's t-test.
   **C.** Knockdown of AdipoRl abrogates ULMA effect on AMPK and p38 signalling. C2C12 myotubes in 6 well plates were transfected with 100nM non-silencing control or AdipoRl siRNAs using DharmaFECT 1 transfection reagent (Thermo). 72h following transfection, cells were treated with vehicle (DMSO) or ULMA (10nM) for 10 min and were western blotted to evaluate pAMPK, pACC, p38, AdipoRl, and R2 status. Total AMPK, ACC, p38 and beta-actin were used as loading controls. Data is representative of three independent experiments displaying identical pattern (Right panel shows densitometry of three experiments). *p<0.05, **p<0.01, ***p<0.001 as determined by two tailed unpaired student's t-test.

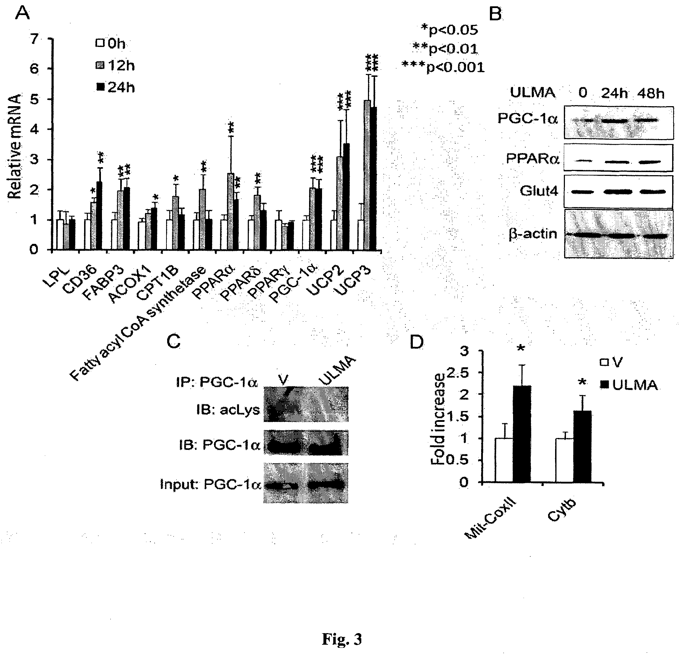

**Fig. 3****: ULMA induces adiponectin target genes, enhances PGC-la expression and activity and increases mitochondrial biogenesis.**
   **A.** ULMA induces expression of fatty acid transport, oxidation, mitochondrial biogenesis and energy dissipation related genes. C2C12 myotubes in 6 well plates were treated with 10nM ULMA or vehicle (designated as 0 h; 0.1% vol/vol DMSO) for 12h or 24h. Following treatment, RNA was extracted and 1 µg RNA from each well was used to prepare cDNA and the cDNAs were then used for quantitative real-time PCR for indicated genes. Beta-actin was used as normalizing control. The relative mRNA level was quantitated using ddCT method and plotted as fold activity over vehicle treated control (0 h). Data is mean ± SEM of 4-6 independent experiments performed in triplicates. *p<0.05, **p<0.01, ***p<0.001 compared to vehicle (DMSO) treated controls as determined by two tailed unpaired student's t-test.
   **B.** ULMA induces protein levels of PGC-1α, PPARα and Glut4. C2C12 myotubes in 10 cm dish were treated with vehicle (DMSO) or 10 nM ULMA in DMSO for 24 or 48 h followed by western blotting with indicated antibodies. Data is representative of three independent experiments showing identical pattern.
   **C.** ULMA induces PGC-la deacetylation. C2C12 myotubes plated in 10 cm dish were treated with vehicle (0.1% vol/vol DMSO) or 10 nM ULMA in DMSO for 6 h. The cell lysates were then incubated with 5 µg anti-PGC-la antibody (Calbiochem) for 12 h at 4°C on a rotating wheel set at 10 RPM. 20 µl of protein A and protein G sepharose beads (Sigma; 1:1) was then added to the solution and the incubation was continued for another 2 h. The tubes were then centrifuged (1000 RPM) for 1 min and the supernatant was discarded. The pellets were washed 7 times. The beads were boiled in 50 µl 2X lammeli buffer for 5min and cooled immediately on ice and following quick spin, the supernatants were resolved by denaturing polyacrylamide gel electrophoresis and western blotted with anti-acetylated lysine (acLys) antibody. Data represent one of three independent experiments displaying identical pattern.
   **D.** ULMA increases mitochondrial DNA content. C2C12 myotubes in 6 well plates were treated with vehicle (0.1% vol/vol DMSO) or 10 nM ULMA (in DMSO) for 72 h. Following which, total cellular DNA was isolated from these cells by standard procedure (using a genomic DNA isolation kit; Macherey Nagel; according to manufacturer's instructions) and the mitochondrial DNA content was measured by QRT-PCR based measurement of mitochondrial coxII (Mit-CoxII) and cytochrome b (Cytb) and normalized with genomic glycerol three phosphate dehydrogenase DNA level. Data represent mean ± SEM from three independent experiments performed in triplicates. *p<0.05 as determined by two tailed unpaired student's t-test.

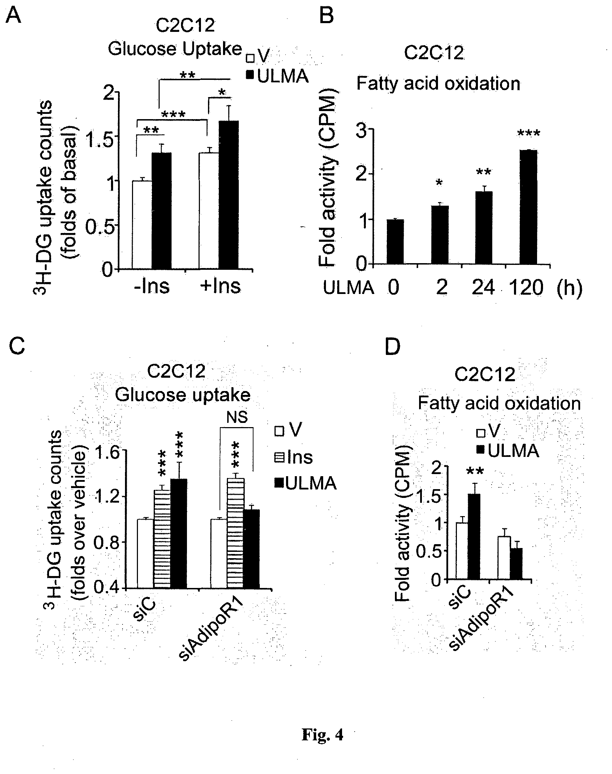

**Fig. 4****: ULMA enhances glucose uptake and fatty acid oxidation in myotubes.**
   **A.** ULMA enhances glucose uptake in C2C12 myotubes. C2C12 myotubes in 24 well plates were treated with vehicle (DMSO) or 10 nM ULMA and glucose uptake assay was performed in presence or absence of 100 nM insulin. Data is mean ± SEM of six independent experiments performed in triplicates. *p<0.05, **p<0.01, ***p<0.001 as determined by two tailed unpaired student's t-test.
   **B.** ULMA enhances fatty acid oxidation in C2C12 myotubes. C2C12 myotubes plated in 12 well plates were treated with vehicle (0.1% vol/vol DMSO) or 10 nM ULMA in DMSO (final concentration of DMSO in all wells 0.1% for 2 h, 24 h or 48 h. Data represent mean ± SEM of three independent experiments performed in triplicates. Following treatment, ¹⁴CO₂ release from these cells was measured and plotted. *p<0.05, **p<0.01, ***p<0.001 as determined by two tailed unpaired student's t-test.
   **C.** siAdipoR1 eliminates ULMA induction of glucose uptake. C2C12 myotubes were transfected with nonsilencing siC or siAdipoR1. 72h after transfection, cells were treated for 24h with 10nM ULMA and glucose uptake assay was performed. Data is mean ± SEM of three independent experiments performed in triplicates. ***p<0.001 as determined by two tailed unpaired student's t-test.
   **D.** siAdipoR1 eliminates ULMA induction of fatty acid oxidation. C2C12 myotubes were transfected with nonsilencing siC or siAdipoR1. 72h after transfection, cells were treated for 24h with 10nM ULMA and fatty acid oxidation experiments were performed. Data is mean ± SEM of three independent experiments performed in triplicates. **p<0.01 as determined by two tailed unpaired student's t-test.

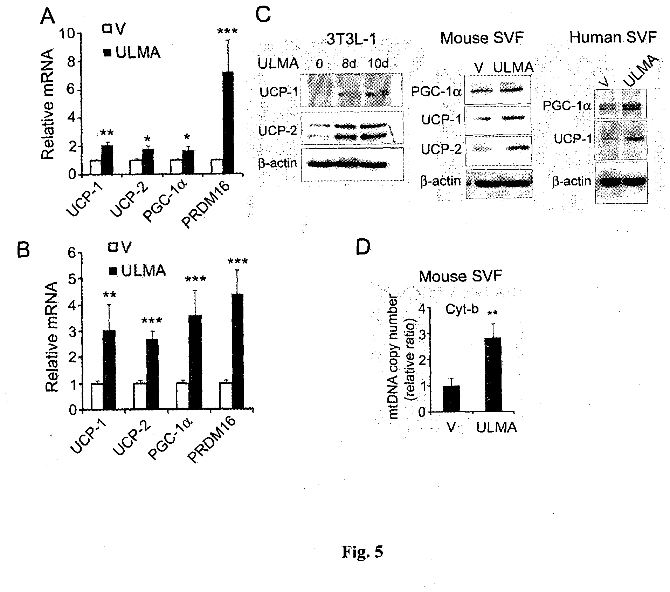

**Fig. 5****: ULMA treatment of 3T3L-1 preadipocytes induces brown adipose marker UCP-1 expression.**
   **A.** 3T3L-1 mouse preadipocyte cells in 6 well plates were allowed to reach full confluence. Two days following confluence (designated as day 0), the growth medium was replaced with differentiation medium (DM). After two days of incubation in DM, this medium was replaced with insulin medium (Ins) and the cells were incubated in Ins for 2 days and then the insulin medium was replaced with growth medium (GM) and the cells were cultured for a total of 10 days (starting from day 0). The cells were treated with vehicle (0.1% DMSO) or ULMA (10 nM in 0.1% DMSO) on day 0 and the total treatment duration was 10 days. In all cases, medium was replaced with fresh corresponding medium every day containing vehicle (0.1 % DMSO) or 10 nM ULMA (in DMSO; final DMSO concentration in all wells 0.1% vol/vol). After a total of 10 days from day 0, cells were washed in cold PBS and RNA was extracted using trizol reagent using standard procedure following which cDNA synthesis was done and UCP-1, UCP-2, PGC-la and PRDM16 expression were determined using QRT-PCR , normalized with Beta-actin mRNA and plotted as fold induction over vehicle treated control. Data is mean ± SEM of three independent experiments performed in triplicates. **p<0.01 as determined by two tailed unpaired student's t-test.
   **B.** Mouse stromal vascular fractions (SVF) isolated from epididymal fat pads using collagenase digestion was differentiated in presence or absence of 10nM ULMA and were analyzed for indicated mRNA expressions. **p<0.01, ***p<0.001 as determined by two tailed unpaired student's t-test.
   **C.** Cells from identical set of experiments as described in figure 5A and 5B or human SVFs prepared by collagenase difgestions were analyzed by western blot analysis for determination of UCP-1, UCP-2 and PGC-la protein level. Beta-actin was used as a loading control. Data is representative of three independent experiments.
   **D.** Mouse SVFs differentiated in presence or absence of 10nM ULMA were evaluated for mitochondrial copy number. Total cellular DNA was isolated from these cells by standard procedure (using a genomic DNA isolation kit; Macherey Nagel; according to manufacturer's instructions) and the mitochondrial DNA content was measured by QRT-PCR based measurement of mitochondrial cytochrome b (Cytb) by qPCR and normalized with genomic glycerol three phosphate dehydrogenase DNA level. Data represent mean ± SEM from three independent experiments performed in triplicates. **p<0.01 as determined by two tailed unpaired student's t-test.

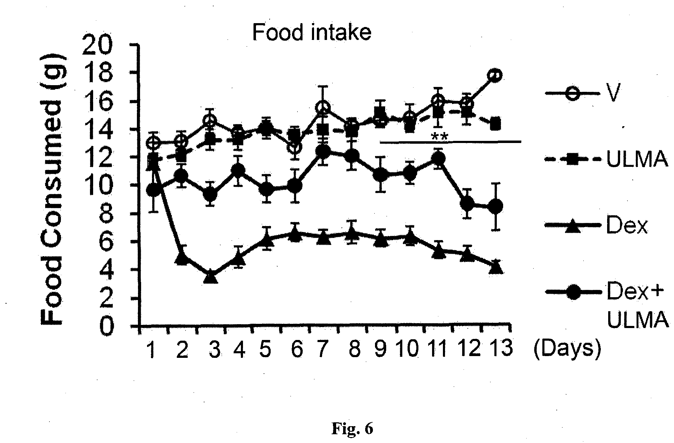

**Fig. 6****: ULMA alleviates dexamethasone-induced reduction of food intake.**
   Six to eight week old wistar rats (n=6 per group) were housed separately and treated as indicated in the example. Daily food intake was measured and plotted. V; 1% gum acacia treated (oral) and 500 µL of 10% ethanol (IP), Dex; 200 µg/kg dexamethasone in 10% ethanol (IP), ULMA; 5 mg/kg ULMA in gum acacia (oral)+ 10% ethanol (IP), Dex+ULMA; 5 mg/kg ULMA (in 1% gum acacia, oral) +200 g/kg dexamethasone (in 10% ethanol, IP). Data represents mean +/- SEM.

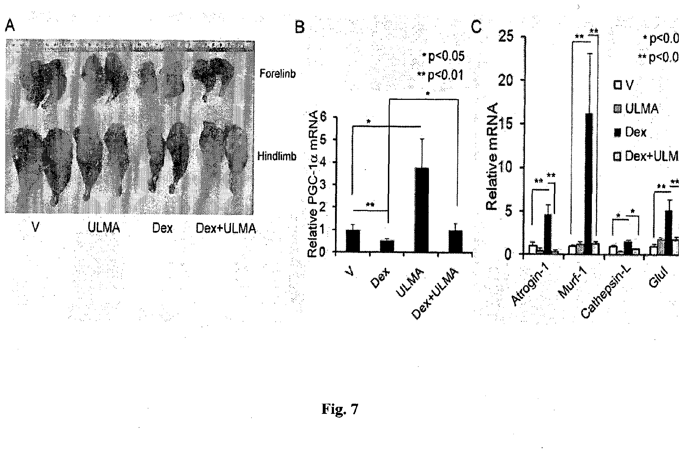

**Fig. 7****: ULMA alleviates dexamethasone mediated induction of atrogene mRNAs.**
   Following 15d indicated treatment of rats, the skeletal muscle (pulled hind limb) were collected and RNA was extracted from them and QRT-PCR was performed in a Roche lightcycler 480 machine. Data represents mean +/- SEM from six animals done in triplicates. "a" statistical analysis between Vehicle and Dexamethasone-treated groups, "b", statistical analysis between dex-treated and dex+ ULMA groups. P<0.05, as determined by student's t-test, n=6.
**Fig. 8****: ULMA reduces dexamethasone mediated increase in heart weight.**
   Following 15d treatment with indicated compounds or vehicle, animals were euthanized and heart weight and body weight were measured and heart weight/body weight ratio were calculated and plotted. Data represents mean +/- SEM. N=6. *p<0.05 as determined by student's t-test.
**Fig. 9****: ULMA alleviates dexamethasone induced glucose intolerance in oral glucose tolerance tests (OGTT).**
   Following 15d treatment with indicated compounds or vehicle, rats were fasted for 16h and then given glucose solution (2g/kg bw) by oral route, and blood glucose was measured by Abott precision xtra glucometer at the indicated time periods. Data represents mean +/- SEM. *p<0.05 between dexamethasone-treated versus dex+ ULMA treated rats. N=6.

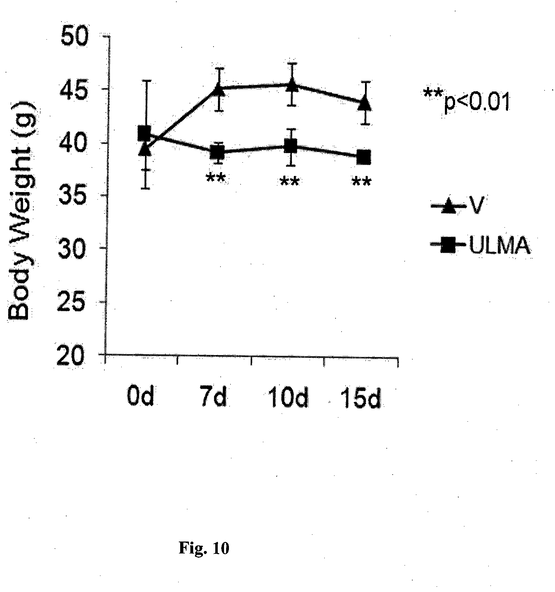

**Fig. 10****: ULMA reduces body weight in db/db mice.**
   8 week old db/db mice were treated with vehicle (V; 1% gum acacia) or with 5 mg/kg ULMA for 15 days and the body weights were measured on 0^{th} , 7^{th}, 10^{th} or 15^{th} day of treatment and plotted. ** p<0.01, n=6.

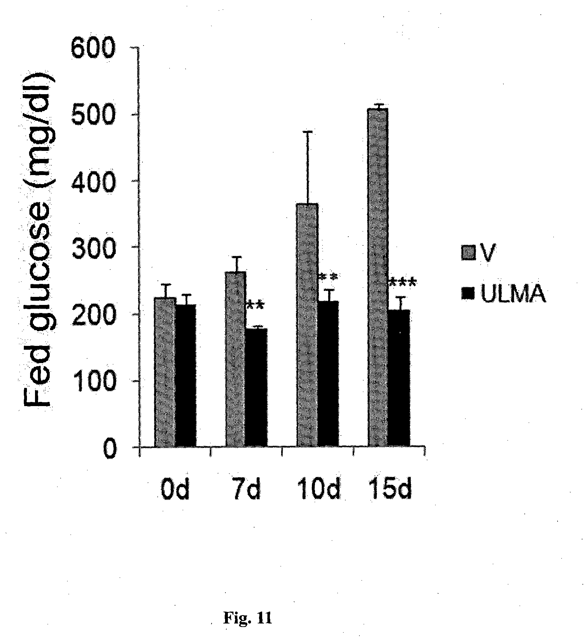

**Fig. 11****: ULMA reduces random blood glucose in db/db mice.**
   8 week old db/db mice were treated with vehicle (V; 1% gum acacia) or with 5mg/kg ULMA for 15 days and the fed blood glucose was measured by a glucometer (Gluco Dr. Super sensor; that is capable of measuring 10-900mg/dl glucose) ** p<0.01, ***p<0.005, n=6.

### DETAILED DESCRIPTION OF THE INVENTION

*Ulmus wallichiana* Planchon was collected from Dehradun and Nainital (State: Uttarakhand, India). ULMA was purified as described earlier [K. Sharan, G. Swarnkar, J. K. Siddigui, A. Kumar, P. Rawat, M. Kumar, G. K. Nagar, L. Manickayasagam, S. P. Singh, G. Mishra, Wahajuddin, G. K. Jain, R. Maurya, N. Chattopadhyay, Menopause 17 (3); 577-586. 2010; R. Maurya, P. Rawat, K. Sharan, J. K. Siddigui, G. Swarnkar, G. Mishra, L. Manickayasagam, G. K. Jain, K. R. Arya, N. Chattopadhyay, WO 2009/110003]. The osteoprotective effects of ULMA has been reported earlier [K. Sharan, G. Swarnkar, J. K. Siddigui, A. Kumar, P. Rawat, M. Kumar, G. K. Nagar, L. Manickayasagam, S. P. Singh, G. Mishra, Wahajuddin, G. K. Jain, R. Maurya, N. Chattopadhyay, Menopause 17 (3); 577-586. 2010; R. Maurya, P. Rawat, K. Sharan, J. K. Siddigui, G. Swarnkar, G. Mishra, L. Manickayasagam, G. K. Jain, K. R. Arya, N. Chattopadhyay, WO 2009/110003]. While seeking for the mechanism through which ULMA exhibits its osteoprotective effects, it was determined that it activated adiponectin receptor signaling. The ULMA in the present invention has been identified as an adiponectin receptor agonist and was evaluated for its effects on adiponectin signaling including induction of glucose-uptake and fatty acid oxidation; and regulation of signaling pathways associated with adiponectin which results in enhancement of glucose uptake and fatty acid oxidation. ULMA was also evaluated for its potential for management, prevention or treatment of metabolic disorders, particularly insulin resistance related disorders caused in humans and animals.

The present invention provides a new use of the compound ULMA as an agonist for adiponectin receptors. Compound ULMA is represented by formula A.

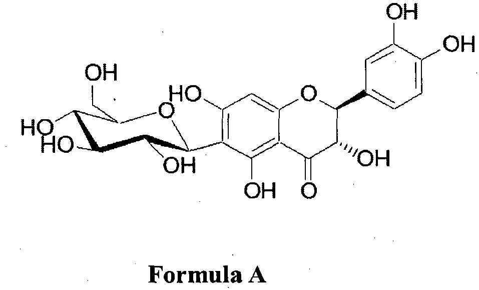

The present invention also provides a method for regulation of adiponectin receptor activity *in vitro* or *in vivo,* wherein *"in vivo"* indicates a human being or any other mammal or an animal within which the regulation of adiponectin receptor activity is required.

The method for prevention or treatment of disorder or a disease condition associated with hypoadiponectimia comprises administering to a subject in need thereof such treatment, a therapeutically effective amount of the compound of the present invention. The subject in need thereof is an animal, preferably a mammal, more preferably a human being.

The present invention also provides a pharmaceutically acceptable salt thereof or a composition comprising a compound of formula A and at least one pharmaceutically acceptable carrier or excipients. The compound of formula A or a composition comprising a compound of formula A can be effectively used *in vitro* (for treatment of cell-lines or primary cells or isolated organ cultures) in the dose ranging from 1 femtomolar to 100 millimolar concentration, preferably from 1 picomolar to 100 micromolar, more preferably from 10 picomolar to 10 micromolar weekly, bi-weekly, daily, twice a day or three times a day or in still more divided doses.

The compound of formula A or a pharmaceutically acceptable salt thereof or a composition comprising a compound of formula A and at least one pharmaceutically acceptable carrier or excipients can be effectively administered in dose from 0.1 mg to 5000 mg, preferably from 0.5 to 1000 mg, more preferably from 1 mg to 500 mg weekly, bi-weekly, daily, twice a day or three times a day or in still more divided doses. The dosage will vary according to the type of disorder or the disease conditions.

Such doses may be administered by any appropriate route selected from the group consisting of oral, systemic, local, topical, intravenous, intra-arterial, intra-muscular, subcutaneous, intra-peritoneal, intra-dermal, buccal, intranasal, inhalation, vaginal, rectal and transdermal..The doses can be in form of a conventional liquid or a solid form to achieve a conventional delivery, a controlled delivery or a targeted delivery of the compound of formula A or a pharmaceutically acceptable salt thereof or a composition comprising the compound of formula A at least one pharmaceutically acceptable carrier or excipient.

The preferred mode of administration of the compound of the present invention or a pharmaceutically acceptable salt thereof or a composition is oral. Oral composition comprises the compound of formula A or a composition comprising the compound of formula A and at least one pharmaceutically acceptable carrier or excipient. The oral composition of the present invention is in the form of tablets, pills, capsules, powders and granules. The liquid composition of the present invention is in the form of a suspension or a liquid formulation. These oral or liquid composition contain at least one of the following pharmaceutically acceptable excipients:
a diluent selected from the group consisting of lactose, mannitol, sorbitol, microcrystalline cellulose, sucrose, sodium citrate and dicalcium phosphate or a combination thereof;
a binder selected from the group consisting of gum tragacanth, gum acacia, methyl cellulose, gelatin, polyvinyl pyrrolidone and starch or a combination thereof;
a disintegrating agent selected from the group consisting of agar-agar, calcium carbonate, sodium carbonate, silicates, alginic acid, corn starch, potato tapioca starch, and primogel or a combination thereof
a lubricant selected from the group consisting of magnesium stearate, calcium stearate , calcium steorotes, talc, solid polyethylene glycols and sodium lauryl sulphate or a combination thereof;
a glidant such as colloidal silicon dioxide ;
a sweetening agent selected from the group consisting of sucrose, fructose and saccharin or a combination thereof;
a flavoring agent selected from the group consisting of peppermint, methyl salicylate, orange flavor and vanilla flavor or a combination thereof;
a wetting agent selected from the group consisting of cetyl alcohol and glyceryl monostearate or a combination thereof;
an absorbent selected from the group consisting of kaolin and bentonite clay or a combination thereof;
a solution retarding agent selected from the group consisting of wax and paraffin or a combination thereof; and a solvent selected from the group consisting of dimethyl sulfoxide, ethanol, methanol and toluene..

The oral composition may contain only pure compound of formula A only.

Further, the present invention seeks to overcome problems associated with the prior art related to cure or management associated with metabolic disorders, particularly insulin resistance related disorders. The present invention also seeks to promote peak bone mass achievement during skeletal growth occurring during adolescence. The ULMA from *Ulmus wallichiana* described in the present invention is useful in management, prevention and treatment of metabolic disorders, preferably in prevention or treatment of insulin resistance disorders caused in humans and animals.

### EXAMPLES

The following examples are given by way of the illustration of the present invention and should not be construed to limit the scope of the present invention.

### Biological evaluation

### Example1

### Direct Interaction of ULMA with adiponectin receptor and induction of AdipoR related signalling events

The potential of ULMA to activate adiponectin receptor signaling was evaluated using a PPARα activation assay. Since adiponectin induces PGC-la expression and activity; and PGC-la is a co-activator for PPAR group of proteins, this strategy has been employed earlier in adiponectin research as a determinant of adiponectin activity (T. Yamauchi, J. Kamon, Y. Ito, A. Tsuchida, T. Yokomizok, S. Kita, T. Sugiyama, M. Miyagishi, K. Hara, M. Tsunodaq, K. Murakamiq, T. Ohteki, S. Uchida,S. Takekawa, H. Waki, N. H. Tsuno, Y. Shibata, Y. Terauchi, P. Froguel, K. Tobe,S. Koyasu, K. Taira, T. Kitamura, T. Shimizuk, R. Nagai, T. Kadowaki. Nature. 423(6941):762-769. 2003). To perform this assay, HEK293 cells (human embryonic kidney cell line, from American Type Culture Collection (ATCC), Cat; CRL-1573) that express endogenous AdipoRl were plated on 24 well plates in DMEM containing 4.5g/L glucose, 4.0mM glutamine, 1 mM sodium pyruvate, 10% FBS and 1X antibiotic-antimycotic solution (all reagents from invitrogen). 24 hours following plating, these cells were transfected with 100ng of GAL-PPARα (PPARα cDNA fused with GAL4 DNA binding domain) and 100ng GAL4 upstream activation sequence driven luciferase (GAL4-Luc) reporter gene that is capable of binding to any protein fused to GAL4-DNA binding domain (in this case GAL-PPARa) and 100ng Green fluorescence expressing plasmid (GFP) that was used as an internal control, using lipofectamine LTX transfection reagent (Invitrogen) according to manufacturers' instructions. 24 hours after transfection, the cells were treated with vehicle (0.1% DMSO) or 100 pM GW-7647 (PPARα agonist) in DMSO, with or without co-treatment of 100nM ULMA or 1µg/ml globular adiponectin for 6 hours. The cells were then lysed and luciferase activity was measured in a Promega GloMax luminometer using steady Glo luciferase assay kit (Promega) according to manufacturers' instructions; and GFP fluorescence was measured in a fluorimeter (Polarstar Galaxy, BMG Labtech). The luciferase values were normalized with GFP values and plotted as fold luciferase activity. The result obtained in provided in Figure 1A. As observed from the figure, GW-7647 alone increased the luciferase activity by 2 folds over vehicle-treated controls. This activity was further enhanced by ULMA and globular adiponectin, although ULMA was more potent than globular adiponectin.

To investigate whether this enhancement of PPAR ligand activity by ULMA indeed happens through AdipoRs, physical interaction between ULMA and AdipoRs were assessed. The ULMA was immobilized on agarose beads.

### Preparation of ULMA Specific Affinity Matrix:

ULMA specific Affinity Matrix and Control Matrix were prepared using following protocol.
1. 45 mg and 22.5 mg of epoxy-activated agarose beads (sigma) were weighed and put separately in two 1.5 ml eppendorf tubes. Tubes were labeled as tube -1 and tube -2.
2. The beads were washed 6X, 1 ml each, with double distilled water (DDW). For washing, 1ml of DDW was added to the tubes and the tubes were vortexed for 2 seconds. Tubes were then centrifuged for 10 seconds using fix angle micro centrifuge. Supernatant was removed and another 1 ml of DDW was added and washing was repeated.
3. The beads were then washed 3X with 50% DMF/0.1M Na₂CO₃ solution.
4. For ULMA affinity matrix: 10 mg of ULMA was weighed and dissolved in 20 µl of DMSO and then 130 µl of 50% DMF/0.1M Na₂CO₃ was added to the solution. This 150 µl of small-molecule solution was then added to the washed beads in tube-1. The beads were then vortexed briefly and NaOH at a final concentration of 10 mM was added to it. Tube was covered with the aluminium foil and left overnight on rotary shaker set at 20 RPM.
5. For Control matrix: 150 µl of 1M Ethanolamine solution in 50% DMF/0.1M Na₂CO₃ was prepared and added to the washed beads in tube-2. The ethanolamine blocks the hydorxyl specific functional groups on the beads and thus a control matrix devoid of any ULMA were prepared. Tube was covered with the aluminium foil and left overnight on rotary shaker set at 20 RPM.
6. Next day, the tubes were centrifuged for 10 seconds and supernatant was collected in a wash tube.
7. Beads were washed 3X with 50 µl of 50% DMF/0.1M Na₂CO₃ solution to remove the trace amounts of uncoupled ULMA.
8. In ULMA specific affinity beads, 300 µl of 1M final concentration of Ethanolamine solution was added to block any remaining reactive hydroxyl group. Tube was covered with aluminium foil and left for 3 hours on rotary shaker set at 20 RPM. Control beads were left untouched over this period.
9. After 3 hours, tube-1 and tube-2 were centrifuged for 10 seconds and supernatant was removed.
10. The beads were then washed 3X with 500 µl of 50% DMF/0.1M Na₂CO₃ solution to remove the unbound ethanolamine.
11. The beads were further washed 6X, 1 ml each, with high salt buffer (1M NaCl, 50 mM HEPES and 0.1% triton)
12. At this stage beads were ready for incubation with protein source.

### For ULMA-AdipoR interaction assay

C2C12 mouse myoblast cells (ATCC, Cat; CRL-1772) were maintained in growth medium (DMEM containing 4.5g/L glucose, 4.0mM glutamine, 1 mM sodium pyruvate, 10% FBS and 1X antibiotic-antimycotic solution (all reagents from Invitrogen)). For differentiation into myotubes, cells were seeded on T75 flasks. When the cells reached visual confluence, the medium was changed to C2C12 differentiation medium (DMEM containing 4.5g/L glucose, 4.0mM glutamine, 1 mM sodium pyruvate, 2% horse serum and 1X antibiotic-antimycotic solution). The cells were maintained in differentiation medium for 4 days when clear myotubes were visualized. 50µg of membrane extracts (prepared using a membrane protein isolation kit; Biomol; according to manufacturer's instructions) prepared from these C2C12 myotubes (that express both AdipoRl and AdipoR2) were incubated with 20µl control or ULMA-beads in 500µl binding buffer (1M NaCl, 50 mM HEPES and 0.1% triton X 100) for 12 hours on a rotary wheel set at 10 rpm. The supernatant was removed and an aliquot was stored as flow-through. The pellet was washed 6 times using 500µl wash buffer (1M NaCl, 50 mM HEPES and 0.5% triton X 100) and then the beads were boiled in Lamelli buffer and resolved by 10% denaturing polyacrylamide gel electrophoresis followed by transfer on nitrocellulose membrane and western blotting with anti AdipoRl or anti-AdipoR2 antibodies (antibody dilution 1:1000) as described earlier (S.K. Dwivedi, N. Singh, R. Kumari, J.S. Mishra, S. Tripathi, P. Banerjee, P. Shah, V. Kukshal, A.M. Tyagi, A.N. Gaikwad, R.K. Chaturvedi, D.P. Mishra, A.K. Trivedi, S. Sanyal, N. Chattopadhyay, R. Ramachandran, M.I. Siddiqi, A. Bandyopadhyay, A. Arora, T. Lundasen, S.P. Anakk, D.D. Moore, S. Sanyal. Mol Endocrinol. 25(6):922-932. 2011). The results are provided in Figure 1B. As observed, both AdipoRl and R2 can be detected on ULMA beads, but not on control beads indicating that ULMA physically interacts with AdipoRs.

### Competitive radio-ligand binding assay

Further validation of AdipoR-ULMA interaction was obtained using a competitive radio-ligand binding assay. C2C12 myotubes in 12 well plates were incubated with 2µl of 10uCi/ml 125I-adiponectin (this amount gives 50% binding to the cells) in ice-cold phosphate buffer saline (PBS; 20mM phosphate, 150mM NaCl, pH7.4) and 0.1% bovine serum albumin for 12 hours in presence or absence of different doses of unlabeled ULMA in DMSO (10⁻¹¹ M, 10⁻¹⁰ M, 10⁻⁹ M, 10⁻⁸ M, 10⁻⁷ M, 10⁻⁶M) at 4°C (final concentration of DMSO in all wells was 0.1% vol/vol). The cells were then washed 20 times in PBS and the cells were lysed in 400µl lysis buffer (0.1N NaOH and 0.1% SDS). 5µl of the lysate was used for protein estimation using standard Bradford assay and rest of the lysate was used for measuring radioactivity in a gamma-counter (Cole Palmer). The count per minute was normalized with protein concentration and plotted as % binding compared to wells treated with vehicle (0.1% DMSO). The results are provided in Figure 1C. Cold ULMA dose-dependently replaced 1251-adiponectin binding to the myotubes indicating that ULMA indeed binds to AdipoRs.

Further validation of AdipoR-ULMA interaction and its quantitation was done using a radio-ligand saturation binding assay using ¹²⁵I-ULMA. ULMA was first radiolabeled using chloramines-T method. 10µl of ¹²⁵I (20 MBq; BARC, Mumbai, India) was added to 100µg GTDF in 5% acetic acid/methanol, then chloramine-T (Sigma, 4µg in MilliQ H₂O) was added, and the mixture was allowed to react at room temperature (24°C) for 5 min. The reaction was terminated by adding 60µl sodium metabisulphite (Sigma, 4mg/ml in MilliQ H₂O). The reaction mixture was dried by passing nitrogen and was dissolved into methanol (100 µl). Reverse phase TLC (RP-18 F254s, Merck, 8cm in length) was used to purify ¹²⁵I-GTDF from free iodine and unlabeled compound using methanol-water (40%-60%) as mobile phase. Following run, the TLC plate was cut into pieces of 0.5mm each and the distribution of radioactivity along the plate was measured in a Gamma Counter. TLC of the blank reaction suggested the location of free ¹²⁵I in the TLC plate. The RF value of the labeled compound was determined by gamma counting. The area showing maximum activity at distance of 40 to 60 mm was eluted from the TLC plate, and was washed with methanol, centrifuged, decanted and dried under N₂ followed by reconstitution in 100 µl DMSO and further dilution in PBS containing 0.1% BSA. For binding assays, Chinese Hamster Ovary cells (Cat: 85050302-1VL, European Collection of Cell Cultures (ECACC); marketed by Sigma) which do not express endogenous AdipoRs were transfected with 500nM of empty vector or AdipoRl or AdipoR2 expression vectors in 24 well plates using Lipofectamine LTX (invitrogen) according to manufacturer's instructions. 24 hours after transfection, cells were incubated with various concentrations of ¹²⁵I-ULMA in ice cold PBS+0.1% BSA for 2 hours (at this time point, binding equilibrium was reached). Cells were then washed with PBS and lysed in 200µl lysis buffer (0.1N NaOH and 0.1%SDS). 5µl of the lysate was used for protein estimation using standard Bradford assay and rest of the lysate was used for measuring radioactivity in a gamma-counter (Cole Palmer). The count per minute was normalized with protein concentration and was further normalized with non specific binding, which was determined for every concentration of ¹²⁵I-ULMA, using 200 fold molar excess of unlabeled ULMA. The result obtained is provided in Figure 1D. As shown in the figure, ¹²⁵I-ULMA failed to show any binding with the empty vector transfected CHO cells, while it showed strong binding with CHO cells over-expressing AdipoRl or R2. Calculated dissociation constant *(Kd)* and maximum binding *(B_{MAX})* of ULMA for AdipoRl were 4.90nM and 1410 fmol/mg of protein respectively, and for AdipoR2, *Kd* and *B_{MAX}* of ULMA were 326 nM and 3950 fmol/mg of protein, respectively.

ULMA was next checked for its ability to regulate signalling pathways that are known to be regulated by adiponectin [T. Kadowaki, T. Yamauchi, N. Kubota, K. Hara, K. Ueki, K. Tobe. J Clin Invest. 116(7): 1784-92. 2006; M. Iwabu, T. Yamauchi, M. Okada-Iwabu, K. Sato, T. Nakagawa, M. Funata, M. Yamaguchi, S. Namiki, R. Nakayama, M. Tabata, H. Ogata, N. Kubota, I: Takamoto, Y. K. Hayashi, N. Yamauchi, H. Waki, M. Fukayama, I. Nishino, K. Tokuyama, K. Ueki, Y. Oike, S. Ishii, K. Hirose, T. Shimizu, K. Touhara, T. Kadowaki. Nature; 464(7293):1313-1319. 2010; T. Yamauchi, J. Kamon, Y. Ito, A. Tsuchida, T. Yokomizok, S. Kita, T. Sugiyama, M. Miyagishi, K. Hara, M. Tsunodaq, K. Murakamiq, T. Ohteki, S. Uchida, S. Takekawa, H. Waki, N. H. Tsuno, Y. Shibata, Y. Terauchi, P. Froguel, K. Tobe, S. Koyasu, K. Taira, T. Kitamura, T. Shimizuk, R. Nagai, T. Kadowaki. Nature. 423(6941):762-769. 2003].

C2C12 myotubes in 10 cm dishes were treated with vehicle (0.1% DMSO; 0 min) or 10 nM ULMA for different time points ranging from 1 min to 24 hour. Following treatment, the cells were washed with ice-cold PBS and then lysed in lysis buffer [1M NaCl, 50 mM HEPES and 0.1% triton X 100 containing 1X protease inhibitor cocktail and 1X phosphates inhibitor cocktail (Sigma)]. The total protein was estimated by Bradford assay and equal amount of protein (50µg) was resolved by denaturing polyacrylamide gel electrophoresis and western blotted for determination of pAMPK, pACC and pP38 levels (in all the cases the primary antibodies were used in 1:1000 dilution and all the antibodies were from Cell signaling technology). Total AMPK, ACC and p38 expressions were also detected and used as loading controls (all the antibodies were from Cell signaling technology and were used in 1:1000 dilution). The result is provided in Figure 1A. As shown in the figure, ULMA caused a rapid and robust phosphorylation of AMPK and its target ACC, it also phosphorylated p38 (The bar chart in right panel of Fig.2A displays quantitation using densitometry). To evaluate if AdipoRs are indeed involved in the regulation of these pathways by ULMA, C2C12 myoblasts growing in T75 flasks were trypsinized and transfected with 10µg of either empty vector (pcDNA3) or AdipoRl expression plasmid using lipofectamine LTX transfection reagent and then the cells were plated in 10 cm dishes. 24 hours following transfection, cells were incubated in differentiation medium and were maintained in the same medium for 96 hours when the cells fully differentiated into myotubes. These cells were then treated with vehicle (DMSO) or ULMA (10nM) for 10 min. The cells were then lysed (as mentioned above) and western blotted. The result obtained in provided in Figure 2B. As shown in the figure, AdipoRl overexpression caused a robust increase in effect of ULMA, indicating that ULMA actions are mediated through AdipoRs (Right panel in Fig. 2B displays quantitation by densitometry). This was further validated in knockdown experiments where C2C12 myotubes in 6 well plates were transfected with 100nM non-silencing control or AdipoRl siRNAs using DharmaFECT 1 transfection reagent (Thermo). 72 hours following transfection, cells were treated with vehicle (DMSO) or ULMA (10nM) for 10 min and were western blotted to evaluate pAMPK, pACC, pP38, AdipoRl, and R2 status. The result obtained is provided in Figure 2C. As shown in the figure 2C, siAdipoR1 successfully down regulated the expression of AdipoRl without affecting AdipoR2 expression; and siAdipoR1 completely obliterated ULMA response on AMPK, ACC and p38 phosphorylation, whereas ULMA phosphorylated AMPK, ACC and P38 in presence of control siRNA (siC). Together, Figure 2 clearly shows that ULMA regulates the adiponectin signaling pathway and this regulation is dependent on AdipoRl.

### Example 2

### Induction of expression of genes responsible for fatty acid transport, oxidation mitochondrial biogenesis and glucose transporter 4 by ULMA

Adiponectin enhances transcription of genes regulating fatty acid transport, fatty acid - oxidation and mitochondrial uncoupling proteins in skeletal muscle and myotubes (T. Kadowaki, T. Yamauchi, N. Kubota, K. Hara, K. Ueki, K. Tobe. J Clin Invest. 116(7): 1784-92. 2006; S. Dridi, M. Taouis. Journal of Nutritional Biochemistry 20 (2009): 831-839. 2009; M. Iwabu, T. Yamauchi, M. Okada-Iwabu, K. Sato, T. Nakagawa, M. Funata, M. Yamaguchi, S. Namiki, R. Nakayama, M. Tabata, H. Ogata, N. Kubota, I. Takamoto, Y. K. Hayashi, N. Yamauchi, H. Waki, M. Fukayama, I. Nishino, K. Tokuyama, K. Ueki, Y. Oike, S. Ishii, K. Hirose, T. Shimizu, K. Touhara, T. Kadowaki. Nature; 464(7293):1313-1319. 2010). The ability of ULMA to influence expression of these genes were checked in c2c12 myotubes. C2C12 myotubes in 6 well plates were treated with 10 nM ULMA or vehicle (DMSO) for 12 hours or 24 hours. Following treatment, the cells were washed with ice-cold PBS and RNA was extracted using Trizol (Ambion) according to manufacturer's instructions. The RNAs were quantitated using a spectrophotometer (nanophotometer; Implen GMBH) and 1µg RNA was used to prepare cDNA using a cDNA synthesis kit (Applied Biosystems). cDNAs were then used for quantitative real-time PCR for indicated genes (Fig. 3A) using Veriquest SYBR green QRT-PCR mastermix (US Biologicals) and a Roche lightcycler 480 thermal cycler (Roche Diagnostics). Beta-actin was used as normalizing control. The relative mRNA level was quantitated using ddCT method. The result obtained is provided in Figure 3A. As shown in the figure, ULMA induced expression of fatty acid transporters CD36 and FABP3. ULMA also enhanced expressions of ACOX1, CPT1B and fatty acyl CoA synthetase (enzymes that regulate fatty acid -oxidation). ULMA induced expression of PPARα and PGC-la, the former a transcription factor and latter a co-activator that are involved in fatty acid oxidation, mitochondrial biogenesis and enhancement of mitochondrial activity. ULMA also induced expressions of uncoupling proteins 2 and 3 in these cells. Adiponectin is also known to induce muscle and adipose glucose transporter 4 (GLUT4) expressions; and the ability of ULMA to induce the expression of GLUT4, PPAR and PGC-la was investigated by western blotting. C2C12 myotubes in 10 cm dish were treated with vehicle (DMSO) or 10nM ULMA in DMSO for 24 hours or 48 hours followed by western blotting as described [S.K. Dwivedi, N. Singh, R. Kumari, J.S. Mishra, S. Tripathi, P. Banerjee, P. Shah,V. Kukshal, A.M. Tyagi, A.N. Gaikwad, R.K. Chaturvedi, D.P. Mishra, A.K. Trivedi, S. Sanyal,N. Chattopadhyay, R. Ramachandran, M.I. Siddiqi, A. Bandyopadhyay, A. Arora, T. Lundasen, S.P. Anakk, D.D. Moore, S. Sanyal . Mol Endocrinol. 25(6): 922-932. 2011). The result obtained in provided in Figure 3B. As shown in the figure, ULMA induced protein levels of PGC-la, PPAR and GLUT4 [PGC-1α antibody from Calbiochem, PPAR and Glut4 antibodies were from cell signal ling technology; all dilutions 1:1000].

### Example 3

### Induction of PGC-la deacetylation and enhancement of mitochondrial biogenesis by ULMA.

Adiponectin is also known to activate PGC-la by indirectly deacetylating this protein through activation of sirt1 (M. Iwabu, T. Yamauchi, M. Okada-Iwabu, K. Sato, T. Nakagawa, M. Funata, M. Yamaguchi, S. Namiki, R. Nakayama, M. Tabata, H. Ogata, N. Kubota, I. Takamoto, Y. K. Hayashi, N. Yamauchi, H. Waki, M. Fukayama, I. Nishino, K. Tokuyama, K. Ueki, Y. Oike, S. Ishii, K. Hirose, T. Shimizu, K. Touhara, T. Kadowaki. Nature; 464(7293):1313-1319. 2010), therefore PGC-la acetylation status following ULMA treatment was checked in C2C12 cells. C2C12 myotubes plated in 10 cm dish were treated with vehicle (DMSO) or 10nM ULMA in DMSO for 6 hours. The cell lysates (500µl; lysis buffer; 1M NaCl, 50 mM HEPES and 0.1% triton X 100 with 1X protease and phosphatase inhibitor cocktail) in 1.5ml microfuge tubes were then incubated with 5µg anti-PGC-1α antibody (Calbiochem) for 12 hours at 4°C on a rotating wheel set at 10RPM. 20µl of protein A and protein G sepharose beads (Sigma; 1:1) was then added to the solution and the incubation was continued for another 2 hours. The tubes were then centrifuged (1000 R.P.M) for 1 min and the supernatant was discarded. The pellets were washed 6 times in washing buffer (1M NaCl, 50 mM HEPES and 0.5% triton X 100), followed by a final wash in 1M NaCl and 50mM HEPES and the beads were boiled in 50µl 2X lammeli buffer (4% SDS; 20% glycerol; 10% 2-mercaptoethanol; 0.004% bromphenol blue) for 5min and cooled immediately on ice and following quick spin the supernatants were resolved by denaturing polyacrylamide gel electrophoresis and western blotted with anti-acetylated lysine (acLys) antibody (Millipore; 1;1000) and western detection was performed with an enhanced chemi-luminescence detection system (Millipore). The same blot was then stripped using a stripping buffer (Millipore) and probed with PGC-la antibody to determine equal loading. The result obtained is provided in Figure 3C. As shown in figure 3C, ULMA decreased the level of acetylated PGC-la, indicating that it does enhance both PGC-la expression and activity. Since increase in PGC-la expression and activity is correlated with enhancement of mitochondrial biogenesis, the ability of ULMA to induce mitochondrial biogenesis was checked. C2C12 myotubes in 6 well plates were treated with vehicle (0.1% DMSO) or 10 nM ULMA (in DMSO) for 72 hours. Following which, total cellular DNA was isolated from these cells by standard procedure (using a genomic DNA isolation kit; Macherey Nagel; according to manufacturer's instructions) and the mitochondrial DNA content was measured by QRT-PCR as described above and normalized with genomic glycerol three phosphate dehydrogenase DNA level. The result obtained is provided in Figure 3D. As shown in the figure, ULMA enhanced mitochondrial cytochrome oxidase II (COX-II) and Cytochrome B (Cytb) levels; indicative of higher mitochondrial content.

### Example 4

### Enhanced glucose uptake and fatty acid oxidation in cultured myotubes by ULMA

Adiponectin is known to enhance glucose uptake and fatty acid oxidation in skeletal muscle and myotubes (T. Kadowaki, T. Yamauchi, N. Kubota, K. Hara, K. Ueki, K. Tobe. J Clin Invest. 116(7): 1784-1792. 2006; T. Yamauchi, J. Kamon, Y. Ito, A. Tsuchida, T. Yokomizok, S. Kita, T. Sugiyama, M. Miyagishi, K. Hara, M. Tsunodaq, K. Murakamiq, T. Ohteki, S. Uchida, S. Takekawa, H. Waki, N. H. Tsuno, Y. Shibata, Y. Terauchi, P. Froguel, K. Tobe, S. Koyasu, K. Taira, T. Kitamura, T. Shimizuk, R. Nagai, T. Kadowaki. Nature. 423(6941):762-769. 2003). Therefore the ability of ULMA to influence insulin-dependent and independent glucose uptake and fatty acid oxidation was investigated in C2C12 myotubes. For glucose uptake assays, C2C12 myotubes in 24 well plates were treated with vehicle (0.1% vol/vol DMSO) or 10nM ULMA (in DMSO; final concentration of DMSO 0.1% in all wells) for 24 hours, following which the cells were maintained in DMEM containing no serum for 3 hours. The cells were then washed three times in warm (37°C) HEPES buffer solution (HBS; 140 mM sodium chloride, 20 mM HEPES, 5 mM potassium chloride, 2.5 mM magnesium sulfate, 1 mM calcium chloride, pH 7.4) and then treated with warm HBS or 100nM insulin (in HBS) for 20 min. Subsequently, cells were washed 3X in warm HBS and then were incubated in 250µl transport solution (HBS containing with 1µCi 3H-deoxyglucose (Perkin Elmer) and 10µM unlabeled 2-deoxyglucose(Sigma)) per well for 5 min. Then, the transport solution was aspirated and the cells were washed 3X with ice-cold stop solution (0.9% NaCl and 25mM dextrose). Subsequently, the cells were lysed in 100µl 0.5N NaOH and 5µl lysate was used for protein concentration determination, and rest of the lysate was used to measure cellular radioactivity in a beta-counter (Beckman Coulter). For fatty acid oxidation experiments, C2C12 myotubes plated in 12 well plates were treated with vehicle (0.1% vol/vol DMSO) or 10nM ULMA in DMSO (final concentration of DMSO in all wells 0.1% for 2h, 24h or 48h). Following treatment, the cells were washed 3X in warm HBS and then incubated with medium containing 0.75 mM palmitate (conjugated to 2% fatty acid free BSA)/14C palmitate at 2 µCi/ml for 2 hours. Following this incubation period, 1 ml of the culture medium was removed and transferred to a sealable tube, the cap of which housed a Whatman (GF/B) filter paper disc that had been presoaked with 1M potassium hydroxide. ¹⁴CO₂ trapped in the media was then released by acidification of media using 60% (vol/vol) perchloric acid and gently agitating the tubes at 37°C for 2 hours. Radioactivity that had become adsorbed onto the filter discs was then quantified by liquid scintillation counting in a beta-counter (Beckman Coulter). The cells were lysed with 200µl 0.5N NaOH and 5µl of the lysate was used for protein estimation using Bradford assay and the radioactivity was normalized with the protein content. The result obtained is provided in Figure 4.

As shown in Figure 4A and B, ULMA enhanced glucose uptake both in presence and absence of insulin and it also robustly induced fatty acid oxidation that was visible within 2 hour of treatment and increased with time. To further assess if ULMA-induction of glucose uptake and fatty acid oxidation were AdipoR-dependent, glucose uptake and fatty acid oxidation experiments were performed in C2C12 myotubes transfected with siC or siAdipoRl; and as shown in Figure 4C and D, siAdipoRl, not siC, completely eliminated ULMA-induced glucose uptake (Fig 4C) and fatty acid oxidation (Fig 4D), while insulin-mediated glucose uptake was unaltered (Fig. 4C).

### Example 5

### Induction of expression of brown adipose tissue markers in adipocytes by ULMA.

Adiponectin has previously been described to enhance mitochondrial function in adipose tissues and induces its thermogenic potential and therefore causes a conversion towards brown adipose phenotype characterized by an increase in UCPs, in particular UCP-1 (I.B. Bauche, S.A.E. Mkadem, A-M. Pottier, M. Senou, M-C. Many, R. Rezsohazy, L. Penicaud, N. Maeda, T. Funahashi, S.M. Brichard. Endocrinology 148(4):1539-1549. 2007). Therefore, the ability of ULMA to induce UCP-1 and 2 in different stages of adipocyte differentiation was checked. We also checked other brown adipose markers such as PGC-la and PR domain containing 16 (PRDM16). 3T3L-1 mouse pre-adipocyte cells (ATCC, CL-173) maintained in growth medium (DMEM with 4.5 mg/ml glucose, 4.0mM glutamine, 1 mM sodium pyruvate, 10% FBS and 1X antibiotic-antimycotic solution (all reagents from invitrogen)) were plated in 6 well plates and allowed to reach full confluence. Two days following confluence, (designated as day 0) the growth medium was replaced with 2 ml of differentiation medium (1.5µg/ml insulin, 0.5mM IBMX and 1.0µM dexamethasone)/well. After two days of incubation in differentiation medium, this medium was replaced with insulin medium (DMEM, 10% FBS, plus 1.5µg/ml insulin) and the cells were incubated in insulin medium for 2 days and then the insulin medium was replaced with growth medium and the cells were then cultured for total of 10 days (from day 0). For ULMA treatment, the cells were treated on day 0 (the day on which differentiation medium was added), and the treatment was continued for a total of 10 days. In all cases, medium was replaced with fresh medium containing vehicle (0.1% DMSO) or 10nM ULMA every day. After 10 days from day 0, cells were washed in cold PBS and RNA was extracted using trizol reagent using standard procedure following which cDNA synthesis was done and transcript expression was determined using QRT-PCR as described above.

Mouse stromal vascular fractions (SVF) from epididymal fat pad were prepared using standard collagenase digestion method. Human SVF was prepared from human lipoaspirates (subcutaneous) collected from an obese individual undergoing liposuction following approval of Institutional Ethical Committee. To isolate SVFs, epidymal fat pads tissue or lipoaspirates were washed 6X in PBS and then were dispensed in tissue culture flasks. 0.2% sterile collagenase (Sigma) solution containing IX antibiotic-antimycotic (Invitrogen) was then added to the adipose and the flasks were shaken vigorously for 10 seconds. The flasks were then incubated at 37°C on a shaker for 2 hours with manual shaking of the flasks for 5-10 seconds every 15 min. After completion of digestion, FBS was added to the final concentration of 10% to the flasks, mixed and the collagenase digested tissue were then dispensed in 50 ml conical tubes and were centrifuged at 400g for 10 min at room temp. The supernatant was discarded and the pellets constituting the SVFs were then reconstituted in culture medium (DMEM/F12 50:50 + 10 % FBS) and plated in T25 tissue culture flasks and cultured for further experiments.

The SVFs were differentiated as for 3T3L-1 (described above), in presence of ULMA or vehicle (10d for mouse SVF and 21d for human SVF), following which they were lysed and used for QPCR or western blot analysis.

For western blot -based determination of UCP-1, UCP-2 and PGC-1α protein level, cells from an identical set of experiment were lysed and western blotted with UCP-1, UCP-2 (Abcam), PGC-la (Calbiochem), or beta-actin (cell signaling technology; used as a loading control) as described above. The result obtained is provided in Figure 5. As shown in the figure , ULMA treatment caused a significant increase in UCP-1, UCP-2, PGC-la and PRDM16 mRNA levels in both 3T3L-1 and mouse SVFs (Figure 5A and B), the protein levels of PGC-la and UCPs were also elevated in 3T3L-1, mouse or human SVFs differentiated in presence of ULMA (Figure 5C). In agreement with higher PGC-la expression and higher oxidative capacity of brown adipose tissue, the mitochondrial DNA copy number, as evidenced by a significantly higher cytb level was also observed.

### Example 6

### Biological evaluation of ULMA in steroid (dexamethasone) induced pathophysiology

All animal experiments were conducted in accordance with current legislation on animal experiments [Institutional Animal Ethical Committee (IAEC)] at C.D.R.I. In all animal experiments, rats were individually housed at 21°C, in 12-h light:12-h dark cycles. All animals had access to normal chow diet and water *ad libitum.*

ULMA induced PGC-la expression in myotubes and enhancement of PGC-la expression in skeletal muscle or myotubes is correlated with protection against skeletal muscle atrophy and overall metabolic fitness, including protection against insulin resistance (T. Wenz, S. G. Rossi, R.L. Rotundo, B.M. Spiegelman, C.T. Moraes. Proc Natl Acad Sci U S A:106(48):20405-20410. 2009; M. Sandri, J. Lin, C. Handschin, W. Yang, Z.P. Arany, S.H. Lecker, A.L. Goldberg, and B.M. Spiegelman. Proc Natl Acad Sci U S A;103(44):16260-16265. 2006), therefore ability of ULMA to prevent synthetic glucocorticoid (dexamethasone)-induced metabolic disorders was evaluated. For this, six to eight week old female wistar rats weighing ∼180-220 gm were divided into four groups (n=8 per group, except for Dex group, in which a total of 18 animals were used). Control group received 1% gum acacia (by oral gavage) and 10% ethanol (500µl, intraperitoneally); Dexamethasone group received 200µg/kg body weight of Dexamethasone in 10% ethanol, intraperitoneally (500 µL); ULMA group received 5mg/kg ULMA in 1% gum acacia by oral gavage and Dex+ULMA group received 200 µg/Kg Dex (Intraperitoneally) and 5mg/kg ULMA (in 1% gum acacia) once daily for 14 days. Food intake was measured daily and the rats were weighed each week. The rats were fasted overnight (O/N) on day 14^{th} and on day 15^{th} oral glucose tolerance test was performed. Following oral glucose tolerance test (OGTT), rats were kept with food and water ad libitum for one day. On day 16^{th}, rats were fasted again O/N and then euthanized. At autopsy, from 5 animals/group, tissues were collected and snap frozen in liquid nitrogen. Blood was collected from cardiac punctures. Plasma was separated from whole blood by centrifugation at 3000 rpm for 20 min immediately after collection of blood and stored at -80°C until further analysis. Forelimb (quadriceps) skeletal muscles were processed for RNA and protein extraction, followed by quantitative real-time PCR (QRT-PCR) analysis. Two animals from each group were used for photography post autopsy.

### Example 6A

### Evaluation of ULMA in dexamethasone induced loss of body weight and death

As demonstrated in table 1, dexamethasone treatment caused loss of body weight and ULMA significantly improved this weight loss following 15 days of treatment. ULMA did not cause any significant change in body weight when given to control animals (data not shown).

**Table 1**

| **Parameters** | **Vehicle** | **Dex** | **Dex+ULMA-1mg** | **Dex+ULMA-5mg** |
|---|---|---|---|---|
| Survival/group (%) | 100 | 46.7 | 73.3 | 100 |
| Initial body weight (g) | 177.9±1.9 | 177.3±2.2 | 178.9±2.4 | 180.7±3.2 |
| Final body weight (g) | 207.7±3.9^{a} | 158.3±4.7^{c} | 176.5±5.9^{c} | 192.7±3.8^{b,c} |

| | | | | |
|---|---|---|---|---|
| **ULMA alleviates dexamethasone induced loss of body weights and protects from dexamethasone-induced death.** Six to eight week old wistar rats (n=10) were treated with vehicle, dexamethasone or indicated doses of ULMA together with dexamethasone for 2 weeks and body weight was measured. Animal numbers were counted at the end of the study. ^{a}P < 0.001 and ^{b}*P* < 0.01 compared to Dex group. ^{c}P < 0.001 compared to vehicle group. | | | | |

### Example 6B

### Biological evaluation of ULMA in dexamethasone-mediated reduction of food intake

Food intake was measured every alternate day by giving measured food to each cage in evening (5.00PM) and collection of residual food in next morning (9.00AM) and measuring it. The residual food was subtracted from the food given and plotted. The result obtained is provided in figure 6. As shown in Figure 6, Dex caused a robust loss in food-intake and ULMA alleviated it. However, ULMA did not affect food intake in vehicle treated (ethanol: IP) rats.

### Example 6C

### Evaluation of ULMA on dexamethasone-induced skeletal muscle atrophy

The result obtained is provided in figure 7. As shown in Figure 7A, denuded forelimb and hindlimb of the rats revealed that the dex group animals has severe abnormality in the limbs including deformed forelimb structures, less muscle content and redness, indicative of skeletal muscle weakness, vascular rupture and bleeding, while ULMA co-treatment prevented it. RNA was isolated from skeletal muscles using Trizol (according to manufacturers' protocol) and reverse transcribed as described above and either PGC-la expression or muscle atrophy related genes (atrogenes) expression were examined. As shown in figure 7B, ULMA caused a robust induction in PGC-la expression. Dexamethasone reduced PGC-la level and this reduction could be protected by ULMA. As shown in figure 7C, dexamethasone robustly induced mRNA levels of Atrogin-1/ Muscle Atrophy F-box (MAFbx), an E3 ubiquitin ligase that mediates proteolysis events that occur during skeletal muscle atrophy, muscle RING-finger protein-1 (MuRF1), another E3 ubiquitin ligases involved in muscle atrophy, Cathepsin L, a lysosomal endopeptidase elevated during muscle atrophy and Glutamate ammonia ligase (Glul), a marker of muscle atrophy (M. Sandri, J. Lin, C. Handschin, W. Yang, Z.P. Arany, S.H. Lecker, A.L. Goldberg, and B.M. Spiegelman. Proc Natl Acad Sci U S A.;103(44):16260-16265. 2006). Treatment with ULMA completely protected the test animals against the induction of these atrogenes by dexamethasone.

### Example 6D

### Evaluation of ULMA on dexamethasone mediated cardiac hypertrophy

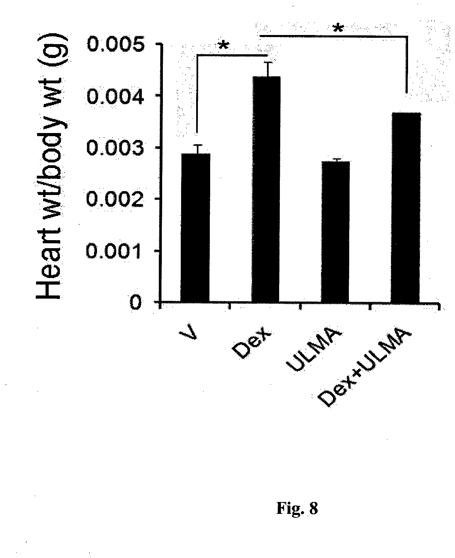

Dexamethasone is known to induce cardiac hypertrophy and enhanced heart weight/body weight ratio is an efficient marker for cardiac hypertrophy. Therefore, the heart weight/body weight ratio was measured in these rats (described in Example 6A-C). The result obtained is provided in Figure 8. As demonstrated in figure 8, dexamethasone enhanced heart/body weight ratio and ULMA significantly alleviated this increase. However, ULMA did not change heart weight/ body weight ratio in control animals.

### Example 6E

### Evaluation of ULMA on Dexamethasone-induced insulin resistance

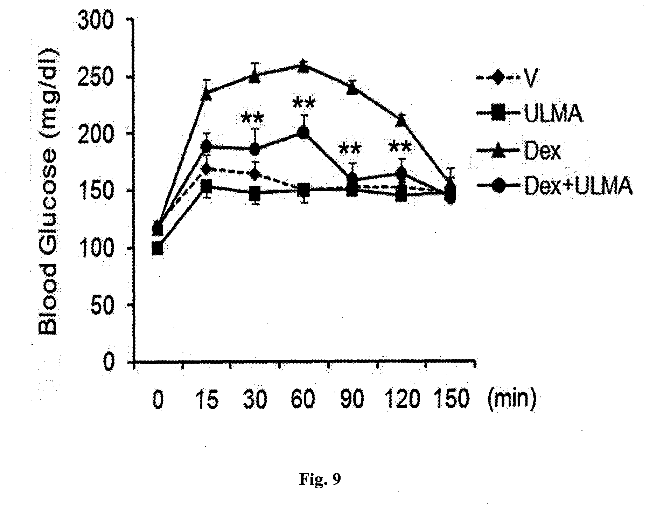

Dexamethasone causes insulin resistance. The ability of ULMA to protect against dexamethasone-mediated insulin resistance was checked. On day 14^{th}, all animals were fasted overnight (water was given ad libitum). The following morning, the rats were given a bolus of glucose (2g/kg body weight), following which blood was collected from tail incision at different time points (0 min, 15 min, 30 min, 60 min, 90 min, 120 min and 150 min) and blood glucose level was measured using a glucometer (Abott precision XTra). The result obtained is provided in Figure 9. As shown in figure 9, dexamethasone treatment caused insulin resistance in rats as evidenced by delayed glucose clearance, while co-treatment with ULMA significantly elevated glucose clearance indicating enhanced insulin sensitivity in these animals. However, ULMA did not affect glucose clearance in control rats.

### Example 6F

### Evaluation of ULMA on dexamethasone-mediated reduction in serum osteocalcin

High dose of dexamethasone has been known to cause osteoblast cell death (B. Espina, M. Liang, R.G. Russell, P.A. Hulley . J Cell Physiol;215(2):488-96. 2008). Osteocalcin is known to be a factor secreted by osteoblasts and is considered as one of the major factors important for maintaining whole body insulin sensitivity and also is implied in pancreatic beta cell survival (A. Neve, A. Corrado, F.P. Cantatore . J Cell Physiol; 228(6):1149-53. 2013). Therefore, in these test animals, the plasma level of osteocalcin was measured using Rat-MID™ Osteocalcin EIA kit (Immunodiagnostics systems) according to manufacturer's instructions. The result obtained is provided in Table 2. As shown in table 2, in dexamethasone treated rats, osteocalcin level was dramatically reduced and this reduction was strongly alleviated in presence of ULMA. Given that increase in osteocalcin level has been implicated in improving insulin sensitivity and also in improving pancreatic beta cell health, ULMA-mediated increase in serum osteocalcin correlates with the improvement of insulin sensitivity in dexamethasone model.

**Table 2:**

| **Group** | **Vehicle** | **Dex** | **Dex +ULMA** |
|---|---|---|---|
| Osteocalcin (ng/ml) | 483.7±19.4^{b} | 263.5±6.4 | 387.7±16.1^{a} |

| | | | |
|---|---|---|---|
| **Table 2. ULMA mitigates dex-induced reduction of serum osteocalcin.** Following 14 days of indicated treatments in wistar rats, serum from the animals were analyzed for osteocalcin level by ELISA. Values are expressed as mean±S.E.M. of 8 independent sets of samples in each treatment group. ^{a}P< 0.05, and ^{b}P<0.001-compared with the Dex treated group. | | | |

### Example 6G

### Evaluation of ULMA on dexamethasone-mediated imbalance in serum Na, K level

Dexamethasone is known to cause hypertension by causing serum sodium (Na), Potassium (K) imbalance. As depicted in table 3, ULMA treatment reversed the elevation of Na and reduction of K caused by dexamethasone.

**Table 3:**

| **Group** | **Control + Vehicle** | **Dex+ Vehicle** | **Dex + ULMA** |
|---|---|---|---|
| Na (mmol/l) | 117±8.8^{b} | 151±2.3 | 124±3.7^{a} |
| K (nmol/l) | 4.04±0.06^{a} | 3.47±0.13 | 3.93±0.08^{a} |

| | | | |
|---|---|---|---|
| **Table 3. ULMA reverses dexamethasone mediated Na, K imbalance in serum.** Following 14 days of indicated treatments in wistar rats, serum from the animals were analyzed for Na and K levels using colorimetric diagnostic kits from Randox Biosystems, India, following manufacturers' instructions. Values are expressed as mean±S.E.M. of 8 independent sets of samples in each treatment group. ^{a}P< 0.05, ^{b}P < 0.01-compared with the vehicle treated Dex group. | | | |

### Example 7

### Biological Evaluation of ULMA in genetically obese and diabetic db/db mice

### Treatment of db/db mice with ULMA

8 week old female db/db mice (n=6) weighing 40-50g were divided into 2 groups and maintained as above. Control groups received vehicle (1% gum acacia) and ULMA groups received 5mg/kg bw ULMA in 1% gum acacia for 7, 10 or 14 days. Body weight and blood glucose were measured at the beginning and end of the studies. All animal experiments were conducted in accordance with current legislation on animal experiments [Institutional Animal Ethical Committee (IAEC)] at C.D.R.I. In all animal experiments, mice were individually housed at 21°C, in 12-h light: 12-h dark cycles. All animals had access to normal chow diet and water *ad libitum.*

### Example 7A

### Evaluation of ULMA on body weight of db/db mice

Since ULMA induced UCP-1 level in adipocytes and PGC-la expression in skeletal muscle and increased fatty acid oxidation and these together indicates that ULMA may enhance metabolic fitness, evaluation of ULMA effect on body weight of genetically obese db/db mice were performed. The result obtained is provided in Figure 10. As demonstrated in Fig 10, db/db mice when treated with 5mg/kg ULMA for 15d had significantly reduced body weight compared to the vehicle (1% gum acacia) treated control mice, indicating that ULMA reduces obesity.

### Evaluation of ULMA on random blood glucose in db/db mice

Along with enhancement of fatty acid oxidation and glucose uptake in skeletal muscles/myotubes, ULMA also enhanced insulin sensitivity in a model of dex-mediated insulin resistance. Therefore, glucose lowering activity of ULMA was observed in db/db mice. The result is provided in Figure 11. As demonstrated in Fig 11, db/db mice when treated with 5mg/kg ULMA for 15d had significantly reduced fed blood glucose in comparison to vehicle (1% gum acacia) treated control mice.

### ADVANTAGE OF THE PRESENT INVENTION

1. ULMA is a small molecule agonist of adiponectin receptor and therefore is superior over adiponectin as adiponectin is a peptide and may have peptide-related stability issues.
2. ULMA does not cause obesity and rather reduces body-weight in obese and diabetic mice.
3. ULMA does not cause hypoglycemia in normal mice.
4. ULMA also maintains serum electrolyte levels and hence may maintain normotensive state.
5. ULMA can ameliorate cardiac hypertrophy.

## Claims

1. A compound of formula A or a pharmaceutically acceptable salt thereof for use in treatment or prevention of adiponectin depletion associated metabolic disorders.

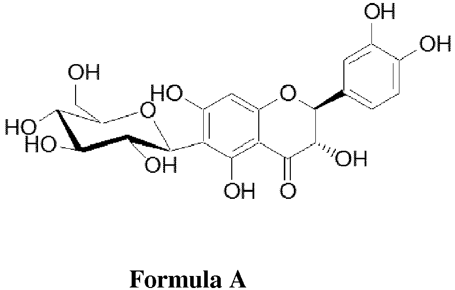

2. The compound according to claim 1 or a pharmaceutically acceptable salt thereof for use in treatment or prevention of adiponectin depletion associated metabolic disorders according to claim 1, wherein said compound is administered in dose from 0.1 mg to 5000 mg, preferably from 0.5 to 1000, more preferably from 1 mg to 500 mg weekly or bi-weekly or daily or twice a day or three times a day or in still more divided doses.

3. The compound according to claim 1 or a pharmaceutically acceptable salt thereof for use in treatment or prevention of adiponectin depletion associated metabolic disorders according to claim 1, wherein said compound is administered by a route selected from the group consisting of oral, systemic, local, topical, intravenous, intra-arterial, intramuscular, subcutaneous, intra-peritoneal, intra-dermal, buccal, intranasal, inhalation, vaginal, rectal and transdermal.

4. The compound according to claim 1 or a pharmaceutically acceptable salt thereof for use in treatment or prevention of adiponectin depletion associated metabolic disorders according to claim 1, wherein said adiponectin depletion associated metabolic disorders is selected from the group consisting of steroid-induced metabolic disorders, skeletal muscle atrophy, induced cardiac hypertrophy and obesity.

5. The compound according to claim 4 or a pharmaceutically acceptable salt thereof for use in treatment or prevention of adiponectin depletion associated metabolic disorders according to claim 4, wherein said steroid is selected from the group consisting of dexamethasone, corticosteroid and prednisolone; said skeletal muscle atrophy is caused by disuse of muscles, denervation, sepsis, fasting or cancer cachexia; and said induced cardiac hypertrophy is selected from the group consisting of neurohormone-mediated hypertrophy, hypoxia-mediated hypertrophy, stress-mediated hypertrophy, myocardial infraction-mediated hypertrophy, hypertension-mediated hypertrophy and drug-induced hypertrophy.

6. A composition comprising a compound of formula A and at least one pharmaceutically acceptable carrier or excipient for use in treatment or prevention of adiponectin depletion associated metabolic disorders.

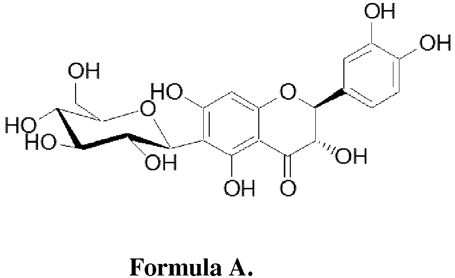

7. The composition according to claim 6 for use in treatment or prevention of adiponectin depletion associated metabolic disorders according to claim 6, wherein said composition is in the form of a suspension, liquid formulation, tablet, pill, capsule, powder or granule containing at least one of the following pharmaceutically acceptable excipient:
(i) a diluent selected from the group consisting of lactose, mannitol, sorbitol, microcrystalline cellulose, sucrose, sodium citrate, and dicalcium phosphate, or a combination thereof;
(ii) a binder selected from the group consisting of gum tragacanth, gum acacia, methyl cellulose, gelatin, polyvinyl pyrrolidone and starch or a combination thereof;
(iii) a disintegrating agent selected from the group consisting of agar-agar, calcium carbonate, sodium carbonate, silicates, alginic acid, corn starch, potato tapioca starch and primogel or a combination thereof;
(iv) a lubricant selected from the group consisting of magnesium stearate, calcium stearate, calcium steorotes, talc, solid polyethylene glycols and sodium lauryl sulphate or a combination thereof;
(v) a glidant such as colloidal silicon dioxide;
(vi) a sweetening agent selected from the group consisting of sucrose, fructose and saccharin or a combination thereof;
(vii) a flavoring agent selected from the group consisting of peppermint, methyl salicylate, orange flavor and vanilla flavor or a combination thereof;
(viii) a wetting agent selected from the group consisting of cetyl alcohol and glyceryl monostearate or a combination thereof;
(ix) an absorbent selected from the group consisting of kaolin and bentonite clay or a combination thereof; and
(x) a solution retarding agent selected from the group consisting of wax and paraffin or a combination thereof.

## Patentansprüche

1. Verbindung der Formel A oder ein pharmazeutisch verträgliches Salz davon zur Verwendung bei der Behandlung oder Vorbeugung von mit Adiponectindepletion verbundenen Stoffwechselstörungen.

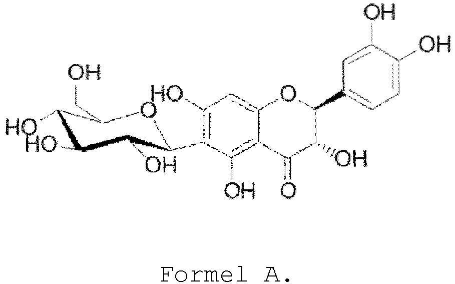

2. Verbindung gemäß Anspruch 1 oder ein pharmazeutisch verträgliches Salz davon zur Verwendung bei der Behandlung oder Vorbeugung von mit Adiponectindepletion verbundenen Stoffwechselstörungen gemäß Anspruch 1, wobei die Verbindung in einer Dosis von 0,1 mg bis 5000 mg, vorzugsweise von 0,5 bis 1000, bevorzugter von 1 mg bis 500 mg wöchentlich oder zweimal wöchentlich oder täglich oder zweimal pro Tag oder dreimal pro Tag oder in noch weiter geteilten Dosen verabreicht wird.

3. Verbindung gemäß Anspruch 1 oder ein pharmazeutisch verträgliches Salz davon zur Verwendung bei der Behandlung oder Vorbeugung von mit Adiponectindepletion verbundenen Stoffwechselstörungen gemäß Anspruch 1, wobei die Verbindung auf einem Weg ausgewählt aus der Gruppe bestehend aus oral, systemisch, lokal, topisch, intravenös, intraarteriell, intramuskulär, subkutan, intraperitoneal, intradermal, bukkal, intranasal, inhalativ, vaginal, rektal und transdermal verabreicht wird.

4. Verbindung gemäß Anspruch 1 oder ein pharmazeutisch verträgliches Salz davon zur Verwendung bei der Behandlung oder Vorbeugung von mit Adiponectindepletion verbundenen Stoffwechselstörungen gemäß Anspruch 1, wobei die mit Adiponectindepletion verbundenen Stoffwechselstörungen ausgewählt sind aus der Gruppe bestehend aus steroidinduzierten Stoffwechselstörungen, Skelettmuskelatrophie, induzierter Herzhypertrophie und Adipositas.

5. Verbindung gemäß Anspruch 4 oder ein pharmazeutisch verträgliches Salz davon zur Verwendung bei der Behandlung oder Vorbeugung von mit Adiponectindepletion verbundenen Stoffwechselstörungen gemäß Anspruch 4, wobei das Steroid ausgewählt ist aus der Gruppe bestehend aus Dexamethason, Corticosteroid und Prednisolon; die Skelettmuskelatrophie durch Nichtgebrauch von Muskeln, Denervierung, Sepsis, Hungern oder Krebskachexie verursacht ist; und die induzierte Herzhypertrophie ausgewählt ist aus der Gruppe bestehend aus neurohormonvermittelter Hypertrophie, hypoxievermittelter Hypertrophie, stressvermittelter Hypertrophie, myokardinfarktvermittelter Hypertrophie, hypertonievermittelter Hypertrophie und arzneimittelinduzierter Hypertrophie.

6. Zusammensetzung, umfassend eine Verbindung der Formel A und wenigstens einen pharmazeutisch verträglichen Träger oder Hilfsstoff zur Verwendung bei der Behandlung oder Vorbeugung von mit Adiponectindepletion verbundenen Stoffwechselstörungen.

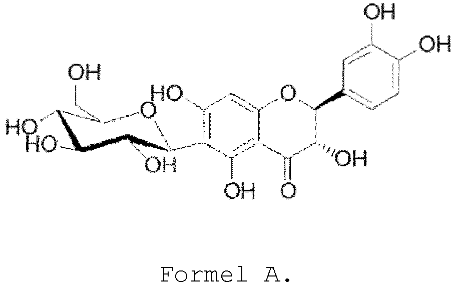

7. Zusammensetzung gemäß Anspruch 6 zur Verwendung bei der Behandlung oder Vorbeugung von mit Adiponectindepletion verbundenen Stoffwechselstörungen gemäß Anspruch 6, wobei die Zusammensetzung in der Form einer Suspension, flüssigen Formulierung, Tablette, Pille, Kapsel, eines Pulvers oder eines Granulats vorliegt, die/das wenigstens einen der folgenden pharmazeutisch verträglichen Hilfsstoffe enthält:
(i) ein Verdünnungsmittel ausgewählt aus der Gruppe bestehend aus Lactose, Mannit, Sorbit, mikrokristalliner Cellulose, Saccharose, Natriumcitrat und Dicalciumphosphat oder eine Kombination davon;
(ii) ein Bindemittel ausgewählt aus der Gruppe bestehend aus Tragantgummi, Akaziengummi, Methylcellulose, Gelatine, Polyvinylpyrrolidon und Stärke oder eine Kombination davon;
(iii) ein Sprengmittel ausgewählt aus der Gruppe bestehend aus Agar-Agar, Calciumcarbonat, Natriumcarbonat, Silicaten, Alginsäure, Maisstärke, Kartoffel-Tapioka-Stärke und Primogel oder eine Kombination davon;
(iv) ein Schmiermittel ausgewählt aus der Gruppe bestehend aus Magnesiumstearat, Calciumstearat, Calciumsteoroten, Talkum, festen Polyethylenglycolen und Natriumlaurylsulfat oder eine Kombination davon;
(v) ein Fließregulierungsmittel, wie z. B. kolloidales Siliciumdioxid;
(vi) ein Süßungsmittel ausgewählt aus der Gruppe bestehend aus Saccharose, Fructose und Saccharin oder eine Kombination davon;
(vii) ein Aromatisierungsmittel ausgewählt aus der Gruppe bestehend aus Pfefferminz, Methylsalicylat, Orangenaroma und Vanillearoma oder eine Kombination davon;
(vii) ein Netzmittel ausgewählt aus der Gruppe bestehend aus Cetylalkohol und Glycerylmonostearat oder eine Kombination davon;
(ix) ein Absorptionsmittel ausgewählt aus der Gruppe bestehend aus Kaolin und Bentonitton oder eine Kombination davon; und
(x) ein lösungsverzögerndes Mittel ausgewählt aus der Gruppe bestehend aus Wachs und Paraffin oder eine Kombination davon.

## Revendications

1. Composé de formule A 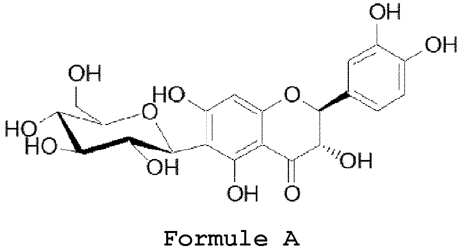 ou sel pharmaceutiquement acceptable de celui-ci destinés à être utilisés en traitement ou prévention de troubles métaboliques associés à un taux réduit d'adiponectine.

2. Composé selon la revendication 1 ou sel pharmaceutiquement acceptable de celui-ci destinés à être utilisés en traitement ou prévention de troubles métaboliques associés à un taux réduit d'adiponectine selon la revendication 1, ledit composé étant administré en une dose de 0,1 mg à 5000 mg, de préférence de 0,5 à 1000, de préférence encore de 1 mg à 500 mg une fois par semaine ou deux fois par semaine ou une fois par jour ou deux fois par jour ou trois fois par jour ou en doses encore plus divisées.

3. Composé selon la revendication 1 ou sel pharmaceutiquement acceptable de celui-ci destinés à être utilisés en traitement ou prévention de troubles métaboliques associés à un taux réduit d'adiponectine selon la revendication 1, ledit composé étant administré par une voie choisie dans le groupe constitué par les voies orale, systémique, locale, topique, intraveineuse, intra-artérielle, intramusculaire, sous-cutanée, intrapéritonéale, intradermique, buccale, intranasale, par inhalation, vaginale, rectale et transdermique.

4. Composé selon la revendication 1 ou sel pharmaceutiquement acceptable de celui-ci destinés à être utilisés en traitement ou prévention de troubles métaboliques associés à un taux réduit d'adiponectine selon la revendication 1, lesdits troubles métaboliques associés à un taux réduit d'adiponectine étant choisis dans le groupe constitué par les troubles métaboliques induits par des stéroïdes, l'atrophie des muscles squelettiques, une hypertrophie cardiaque induite et l'obésité.

5. Composé selon la revendication 4 ou sel pharmaceutiquement acceptable de celui-ci destinés à être utilisés en traitement ou prévention de troubles métaboliques associés à un taux réduit d'adiponectine selon la revendication 4, ledit stéroïde étant choisi dans le groupe constitué par la dexaméthasone, un corticostéroïde et la prednisolone ; ladite atrophie des muscles squelettiques étant provoquée par l'inaction de muscles, une dénervation, un sepsis, le jeûne ou la cachexie cancéreuse ; et ladite hypertrophie cardiaque induite étant choisie dans le groupe constitué par une hypertrophie médiée par des neurohormones, une hypertrophie médiée par une hypoxie, une hypertrophie médiée par le stress, une hypertrophie médiée par un infarctus du myocarde, une hypertrophie médiée par l'hypertension et une hypertrophie induite par un médicament.

6. composition comprenant un composé de formule A 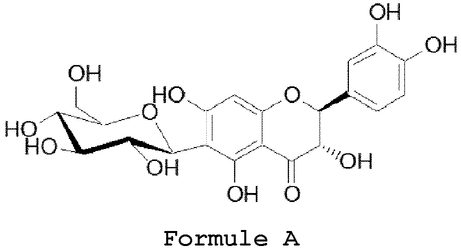 et au moins un vecteur ou excipient pharmaceutiquement acceptable destinée à être utilisée en traitement ou prévention de troubles métaboliques associés à un taux réduit d'adiponectine.

7. Composition selon la revendication 6 destinée à être utilisée en traitement ou prévention de troubles métaboliques associés à un taux réduit d'adiponectine selon la revendication 6, ladite composition étant sous la forme d'une suspension, d'une formulation liquide, d'un comprimé, d'une pilule, d'une capsule, d'une poudre ou d'un granule contenant au moins l'un des excipients pharmaceutiquement acceptables suivants :
(i) un diluant choisi dans le groupe constitué par le lactose, le mannitol, le sorbitol, la cellulose microcristalline, le saccharose, le citrate de sodium et le phosphate dicalcique ou une association de ceux-ci ;
(ii) un liant choisi dans le groupe constitué par la gomme adragante, la gomme arabique, la méthylcellulose, la gélatine, la polyvinylpyrrolidone et l'amidon ou une association de ceux-ci ;
(iii) un agent délitant choisi dans le groupe constitué par l'agar-agar, le carbonate de calcium, le carbonate de sodium, les silicates, l'acide alginique, l'amidon de maïs, l'amidon de tapioca de pomme de terre et le Primogel ou une association de ceux-ci ;
(iv) un lubrifiant choisi dans le groupe constitué par le stéarate de magnésium, le stéarate de calcium, les stéorotes calciques, le talc, les polyéthylèneglycols solides et le laurylsulfate de sodium ou une association de ceux-ci ;
(v) un agent de glissement tel que le dioxyde de silicium colloïdal ;
(vi) un agent édulcorant choisi dans le groupe constitué par le saccharose, le fructose et la saccharine ou une association de ceux-ci ;
(vii) un agent aromatisant choisi dans le groupe constitué par la menthe poivrée, le salicylate de méthyle, l'arôme d'orange et l'arôme de vanille ou une association de ceux-ci ;
(viii) un agent mouillant choisi dans le groupe constitué par l'alcool cétylique et le monostéarate de glycéryle ou une association de ceux-ci ;
(ix) un absorbant choisi dans le groupe constitué par les argiles kaolin et bentonite ou une association de celles-ci ; et
(x) un agent de retardement de mise en solution choisi dans le groupe constitué par la cire et la paraffine ou une association de celles-ci.
